(19) Europäisches Patentamt
European Patent Office
Office européen des brevets

(11) **EP 4 722 229 A1**

(12) **EUROPEAN PATENT APPLICATION**

(43) Date of publication:
**08.04.2026 Bulletin 2026/15**

(21) Application number: **24204326.3**

(22) Date of filing: **02.10.2024**

(51) International Patent Classification (IPC):
***C07K 7/06*** *(2006.01)* ***A61K 38/00*** *(2006.01)*
***A61K 38/12*** *(2006.01)* ***C07K 14/655*** *(2006.01)*
***C07K 14/705*** *(2006.01)*

(52) Cooperative Patent Classification (CPC):
**C07K 7/06; A61K 38/00; A61K 38/12;**
**A61K 40/4202; C07K 14/7051; C12N 5/0636;**
C07K 14/655; C07K 14/705; C07K 2319/03;
C12N 2501/999; C12N 2502/30; C12N 2510/00;
C12N 2740/16043

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
- **Universität Zürich**
  **8006 Zürich (CH)**
- **Eidgenössische Technische Hochschule Zürich**
  **8092 Zürich (CH)**

(72) Inventor: **The designation of the inventor has not yet been filed**

(74) Representative: **Stolmár & Partner**
**Patentanwälte PartG mbB**
**Blumenstraße 17**
**80331 München (DE)**

# (54) ADAPTOR MOLECULES FOR TARGETED CELL IMMUNOTHERAPIES

(57) The invention relates to an adaptor molecule for targeting a cancer cell, comprising a somatostatin receptor 2 (SSTR2) targeting moiety, a linker moiety, and a tag moiety, wherein the SSTR2 targeting moiety is connected to the tag moiety by means of the linker moiety.



Processed by Luminess, 75001 PARIS (FR)

## Description

### Field of the invention

The invention relates to adaptor molecules capable of engaging effector-cells and enhancing the immune response against cells expressing somatostatin receptor type 2.

### Technical background

Neuroendocrine tumors (NETs) are neoplasms arising from cells of the endocrine and nervous systems. In the United States, the age-adjusted incidence rate increased 6.4-fold from 1973 (1.09 per 100 000) to 2012 (6.98 per 100 000). Currently, there are about 12 000 new cases diagnosed each year and 171 000 patients living with Neuroendocrine tumors (cancer.net) which now represent the second most prevalent malignancy of the gastro-entero-pancreatic (GEP) tract.

Somatostatin receptor type 2 (SSTR2) is one of five subtypes of somatostatin receptors and is overexpressed on the surface of most gastro-entero-pancreatic (GEP) NETs, pituitary tumors, paraganglioma, meningioma, as well as hepatocellular carcinoma and breast cancer. Somatostatin receptors (SSTRs) are a family of G protein-coupled receptors that are expressed on the surface of various cells, including those of the endocrine and nervous systems. Therefore, SSTR2 is a promising target for the development of therapies in NETs.

Current targeting tools against SSTR2 are being employed for both diagnostic and therapeutic applications. DOTA-$Tyr^3$-octreotate (DOTA-TATE) is a cyclic peptide agonist, which binds with a high affinity to SSTR2 and SSTR5. Accordingly, specific labeling with either diagnostic or therapeutic isotopes have been explored. [68Ga]-DOTA-TATE has been used for the detection of SSTR2-positive tumors such as GEP-NETs and meningioma. Also, the application of local irradiation with [177Lu]-DOTA-TATE has shown high specificity and efficacy in slowing the progression of NETs in clinical trials.

Several immunological tools have been assessed against NETs using SSTR2 as tumor-associated antigen (TAA). For example, a novel antibody drug conjugate (ADC), consisting of a mAb IgG1 fused via a chemical linker to monomethyl auristatin E (MMAE) was developed. Using this agent in a clinical study, a significant tumor growth retardation was shown. Another example is an anti-SSTR2 x anti-CD3 bispecific antibody including a full Fc domain to extend the serum half-life. The bispecific antibody demonstrated potent anti-tumor activity in subcutaneous NSG in vivo models. Furthermore, the bispecific antibody was shown to activate CD4 and CD8 T-cell activation in cynomolgus monkeys.

Another immunotherapeutic option against SSTR2-positive NETs might be chimeric antigen receptor T-cells (CAR T-cells). CAR T-cells are T-cells expressing, via genetic introduction, a synthetic T-cell activating receptor (frequently derived from an antibody) against defined antigens. The approved application of CAR T-cells is currently limited to B-cell and plasma-cell malignancies, targeting CD19 and B-cell maturation antigen (BCMA). In a recent preclinical study, the efficacy of anti-SSTR CAR T-cells against NETs cell lines was tested. The construct employed two octreotide peptides as SSTR binding moieties, conveying the ability to bind to multiple members of the SSTR family (SSTR2 and SSTR5), and tumor growth retardation in subcutaneous NET xenograft mouse models was demonstrated.

However, the current treatment options for advanced-stage NETs are limited and clinical outcomes are poor. Thus, novel therapeutic approaches are required. While CAR T-cells show high efficacy in some settings, it is also becoming evident that they can induce severe side-effects, e.g., cytokine release syndrome (CRS), making it desirable to develop on-off regulation of CAR T-cell activity. Several suicide gene mechanisms have been developed to eradicate the CAR T-cells in case of severe adverse events, which, however, are also associated with terminal CAR T-cell loss. The need for functional on-off regulation of CAR T-cells has led to the development of adaptor-CAR T-cells (Ad-CAR T-Cells). In this setting, Ad-CAR T-cells bind to a tag, which is placed on the tumor-associated antigen binding agent, composed of an antibody construct or a small molecule ligand. By doing so, the interaction between the Ad-CAR T-cells and the tumor cells depends on the bispecific adaptor's presence. However, functional on-off regulation of Ad-CAR T-cells remains challenging and novel approaches are needed that can provide therapeutic applications against SSTR2-expressing cancer cells.

One of the major hurdles for cell-surface molecule targeting immunotherapies in oncology is tumor-cell specificity. Currently approved, efficient immunotherapies do not sufficiently discriminate between healthy cell-of-origin tissue and malignant cells. Indeed, clinically approved CAR T cells that target highly B- and plasma-cell lineage-specific markers, such as CD19 and BCMA in B- and plasma cell malignancies, ablate healthy B- and plasma cells, leading to B-cell aplasia and hypogammaglobulinemia. A similar approach of tissue-specific antigen targeting in solid tumors would lead to organ ablation and would only be clinically acceptable if the respective organ is not vital.

### Objective problem to be solved

To address this challenge, adaptor molecules are required which can function as a versatile and tunable on-off activating bridge between effector-cells and SSTR2 expressing target cells, thereby regulating effector-cell biocidal

activity while decreasing terminal effector-cell loss, while showing high target specificity and accessibility, in parallel to being safe and of low material cost.

**Summary of the invention**

In one aspect, the invention relates to an adaptor molecule for targeting a cancer cell, the adaptor molecule comprising a somatostatin receptor 2 (SSTR2) targeting moiety, a linker moiety, and a tag moiety, wherein the SSTR2 targeting moiety is connected to the tag moiety by means of the linker moiety.

In a second aspect, the invention relates to an adaptor molecule according to the invention for use in the treatment of a tumor or cancer, wherein the treatment comprises administration of the adaptor molecule and the administration of immune effector cells.

In a third aspect, the invention relates to a method for visualizing a cancer cell, the method comprising targeting the cancer cell with the adaptor molecule according to the invention.

**Brief description of the figures**

**Fig. 1** shows the general synthesis of Octo-Fluo and *in vitro* validation in combination with AdFITC(E2)-CAR T-cells.

**Fig. 1A** shows the chemical structure and one-pot synthesis of compound of formula (III) (Octo-Fluo). Reaction conditions a) DMSO, TEA (10 eq.), 1h, 37°C; b) 1-(2-Aminoethyl)piperidine (10 eq.), 15', 37°C; c) DMSO, 15', 37°C; d) formic acid (up to pH ~ 3), HPLC purification.

**Figs. 1B,** C show an Octo-Fluo titration on Bon1-SSTR2 mCherry Luc cell line, analyzed by flow cytometry, shown as histogram (n=3) (Fig. 1B) and as sigmoid curve (Fig. 1C).

**Fig. 1D** shows immunofluorescence on healthy pancreas tissue and three Paraganglioma patient samples performed using the Octo-Fluo adaptor (scale indicates 100 μm). The green color (fluorescein) and blue (DAPI) represents SSTR2 expression and cell nuclei, respectively.

**Fig. 1E** shows confocal microscopy pictures after exposure to the Octo-Fluo on Bon1-WT and Bon1-SSTR2 expressing cells. The colors represent the following: Green = Octo-Fluo, Red = mCherry (intracellular), Blue = DAPI (nuclear stain), and the scale bar = 10 μM.

**Fig. 1F** shows a graphical representation of Octo-Fluo-dependent bridging of AdFITC(E2)-CAR T-cell to the SSTR2-expressing target cell.

**Fig. 2** shows that AdFITC(E2)-CAR T-cells are fully activated *in vitro* only in presence of both, antigen-expressing target cells and Octo-Fluo adaptor. Quantification of the AdFITC(E2)-CAR T-cell biocidal activity, CD25, CD69 and PD1 expression towards Bon1-SSTR2 mCherry-Luc cells in the presence of increasing concentrations of Octo-Fluo at the indicated time-points 24h (Fig. 2A), 48h (Fig. 2B), and 72h (Fig. 2C) (n= 3 donors). The effector-to-target ratio was fixed at 1:1. The negative controls to all experiments were as follows: NI; AdFITC(E2)-CAR T-cell with 5 nM Octo-Fluo against Bon1-WT mCherry-Luc cells, NII; AdFITC(E2)-CAR T-cells on Bon1-SSTR2 mCherry-Luc without Octo-Fluo added, NIII; Bon1-SSTR2 with 3μM Octo-Fluo. For the statistical analysis, the cell death of each condition was compared to NI.

**Fig. 2D** shows representative flow cytometry plots illustrating AdFITC(E2)-CAR T-cell activation as determined by CD25, CD69 and PD1 expression as well as by cell division (n= 1 donor in triplicate). Effector-cells were co-cultured with tumor cells which were SSTR2 negative or positive, in the presence and absence of the Octo-Fluo and analyzed after 48hr. The effector-to-target ratio was fixed at 1:1. On the right, graphical quantification of the CD25 and CD69 markers as well as PD1 shown in Fig. 2D after 24h, 48h, and 72h.

**Fig. 2E** shows IFNγ and IL-2 quantification analysis by ELISA on the supernatant of the activation assay shown in Fig. 2D. For the statistical analysis, not shown = not significant, **** $P \leq 0.0001$, *** $P \leq 0.0002$, ** $P \leq 0.003$, * $P \leq 0.01$ (two-way ANOVA test, followed by Bonferroni's multiple comparison test).

**Fig. 3** shows AdFITC(E2)-CAR T-cells in combination with Octo-Fluo eliminate established Bon1-SSTR2 mCherry-Luc tumors *in vivo.*

**Fig. 3A** shows a schematic representation of the *in vivo* therapy set-up. At day 0, NSG mice were injected s.c. in the flank with $8\times10^6$ Bon1-SSTR2 mCherry-Luc positive cells. Tumors reached $\pm80mm^3$ on day 14 and were detectable by bioluminescence (IVIS); mice subsequently received $10^7$ AdFITC(E2)-CAR T-cells i.v. and either none or 2.5 nmol or 0.5 nmol of Octo-Fluo every 12h i.v. for 3 weeks; mice were sacrificed at day 35 (n= 3 mice per group).

**Fig. 3B** shows bioluminescence imaging of mice prior to treatment and at the indicated time-points.

**Fig. 3C** shows a graphical quantification of the total IVIS flux measured in the analysis shown in Fig. 3B.

**Fig. 3D** shows the tumor volume and mouse body weight change during the experiment. For the statistically analysis, not shown = not significant, **** $P \leq 0.0001$ (two-way ANOVA test, followed by Bonferroni's multiple comparison test).

**Fig. 4** shows Octo-Fluo dose-dependent *in vivo* anti-tumor efficacy of AdFITC(E2)-CAR T-cells.

**Fig. 4A** shows a schematic representation of the dose escalation study. On day 0, NSG mice were injected s.c. in the flank with 8 x $10^6$ Bon1-SSTR2 mCherry-Luc positive cells. On day 14 (at a tumor volume of $\pm80mm^3$), $10^7$ AdFITC(E2)-CAR T-cells were injected i.v. followed by either 12.5 nmol, 2.5 nmol, or 0.5 nmol Octo-Fluo i.v. injections repeatedly every 12 hours. PBS-injected mice and AdFITC(E2)-CAR T-cell (without Octo-Fluo) injected mice served as negative control. Mice (blood, spleen and tumor) were terminally analyzed at day 28 (n= 3 mice per group). For the statistical analysis, not shown = not significant, **** $P \leq 0.0001$, *** $P \leq 0.0002$, ** $P \leq 0.003$, * $P \leq 0.01$ (two-way ANOVA test, followed by Turkey's multiple comparison test).

**Fig. 4B** shows the percentage of AdFITC(E2)-CAR T-cells detected by flow cytometry ($huCD45^+$) in blood, spleen and tumor tissues of mice at day 28.

**Fig. 4C** shows flow cytometry and immunofluorescence (huCD3) data showing the human immune cell infiltration in tumors at day 28 (scale indicates 100 $\mu$m).

**Fig. 5A** shows the HPLC trace registered at $\lambda$ = 260 nm of intermediate I2.

**Fig. 5B** shows the MS spectra (positive mode) of compound I2.

**Fig. 6A** shows the HPLC trace registered at $\lambda$ = 260 nm of intermediate I2-DBCO.

**Fig. 6B** shows the MS spectra (positive mode) of intermediate I2-DBCO.

**Fig. 6C** shows the MS spectra (negative mode) of intermediate I2-DBCO.

**Fig. 7A** shows the HPLC trace registered at $\lambda$ = 260 nm of compound I.

**Fig. 7B** shows the MS spectra (positive mode) of compound I.

**Fig. 7C** shows the MS spectra (negative mode) of compound I.

**Fig. 8A** shows the HPLC trace registered at $\lambda$ = 260 nm and of crude intermediate I4 (Octo-Azido Linear).

**Fig. 8B** shows the MS spectra (positive mode) of crude intermediate I4 (Octo-Azido Linear).

**Fig. 9A** shows the HPLC trace registered at $\lambda$ = 260 nm of compound II (Octo-Azido cyclized).

**Fig. 9B** shows the MS spectra (positive mode) of compound II (Octo-Azido cyclized).

**Fig. 10A** shows the HPLC trace registered at $\lambda$ = 260 nm of intermediate I5.

**Fig. 10B** shows the MS spectra (negative mode) of intermediate I5.

**Fig. 10C** shows the MS spectra (positive mode) of intermediate I5.

**Fig. 10D** shows the HPLC trace of reaction after quenching the unreacted fluorescein with 1-(2-Aminoethyl) piperidine.

**Fig. 11A** shows the HPLC trace registered at $\lambda$ = 260 nm of compound III (Octo-Fluo).

**Fig. 11B** shows the MS spectra (negative mode) of compound III (Octo-Fluo).

**Fig. 12** shows representative flow cytometry plots of T-cells illustrating the CD4 and CD8 populations, as well as the transduction efficiency (measured using anti-CD34 antibody stain for RQR8 detection). Numbers at gates indicate percentages of cell fraction in each gate.

**Fig. 13A-B** shows representative flow cytometry plots illustrating the negative controls including both T-cells and AdFITC(E2)-CAR T-cell activation as determined by CD25, CD69 and PD1 expression as well as by cell division (n= 1 donor in triplicate). Plots show effector cells exposed to indicated conditions with tumor cells which were SSTR2 negative or positive, in the presence and absence of the Octo-Fluo after 48hrs. The effector-to-target ratio was fixed at 1:1. On the right, quantification of the CD25 and CD69 markers as well as PD1 shown in D after 24h, 48h, and 72h.

**Fig. 14A** shows a schematic representation of the AdFITC(E2)-CAR T-cell in vivo biodistribution experiment. Briefly, NSG mice were injected subcutaneously with 8 x $10^6$ Bon1-SSTR2 cells. At day 14, when the tumor size reached roughly $80mm^3$, we injected both the AdFITC(E2)-CAR T-cells and the Octo-Fluo. The mice received a dose of Octo-Fluo i.v. every 12hr. At day 28, we sacrificed the mice and ran flow cytometry analysis on blood, spleen and tumor samples.

**Fig. 14B** shows the quantification of tumor volumes measured by caliper over the course of the experiment.

**Fig. 14C** shows the measurement of body weight change over time. For the statistical analysis, not shown = not significant, * $P \leq 0.01$ (one-way ANOVA test, followed by Bonferroni's multiple comparison test).

**Fig. 15** shows the principle of effector cell engagement, wherein the tag moiety of the adaptor molecule is a binding target for another bispecific effector-cell engaging molecule.

## Detailed description of the invention

In one aspect, the invention relates to an adaptor molecule for targeting a cancer cell, comprising a somatostatin receptor 2 (SSTR2) targeting moiety, a linker moiety, and a tag moiety, wherein the SSTR2 targeting moiety is connected to the tag moiety by means of the linker moiety.

As used herein, the term "targeting" refers to the ability of a moiety to direct and physically bind to a biological target.

As used herein, the term "effector-cell" refers to any immune effector-cell capable of mediating or effecting an immune response or a cytotoxic response.

The adaptor molecule functions as an on-off tunable activating bridge between effector cells and SSTR2 expressing target cells, thanks to the SSTR2 targeting moiety and the tag moiety, which are located at opposite ends of the adaptor molecule. The SSTR2 targeting moiety binds to SSTR2-expressing tumor or cancer cells, whereas the tag moiety in turn targets effector cells. Different types of effector cells may be provided with an antigen receptor that shows affinity for the specific tag moiety of the adaptor molecule. The linker moiety is the structural link between the SSTR2 targeting moiety and the tag moiety. Different linker moieties may vary in stability, thus impacting the overall stability and half-life of the adaptor molecule, as well as its affinity for the desired antigen.

In one embodiment, the adaptor molecule is a small molecule with a molecular weight of $\leq$ 1000 Da. Small molecules show increased accessibility to the site of action compared to antibody-based molecules and allow a finer control of effector-cell therapies. Due to their small size, the risk of developing an antibody-based antigen-response is lower, as their half-life is very short (of about 30 minutes) compared to long-lived antibodies. This also ensures a tighter control over the immune effector-cell activity, as cessation of administration rapidly results in detachment of the effector-cells from the target cells. In addition, the material cost of small molecule-based compared to antibody-based adaptor molecules is substantially reduced, with a 10-fold difference when it comes to GMP-grade production for clinical investigations.

The SSTR2 targeting moiety of the adaptor molecule is responsible for directing the adaptor molecule to SSTR2.

SSTR2 is one of five subtypes of somatostatin receptors and is overexpressed on the surface of most gastro-entero-pancreatic (GEP) NETs, pituitary tumors, paraganglioma, meningioma, as well as hepatocellular carcinoma and breast cancer.

In a preferred embodiment, the SSTR2 targeting moiety has a high affinity for its target. Thus, the adaptor molecule shows high affinity for and is capable of binding to SSTR2. In a preferred embodiment, the dissociation constant ($K_D$) of the adaptor molecule towards SSTR2 is below 10 nm. In a more preferred embodiment, the $K_D$ of the adaptor molecule is below 5 nm. In a most preferred embodiment, the $K_D$ of the adaptor molecule is below 1 nm. The ideal $K_D$ value is subnanomolar, as above these values small molecules may exhibit unspecific binding to other secondary proteins. Ways to determine $K_D$ value are known to the skilled person, such as Biacore experiments.

In one embodiment, the SSTR2 targeting moiety is a peptide. Peptides can be easily manufactured and are generally well tolerated when administered to humans.

In another embodiment, the SSTR2 targeting moiety is an antibody-based binder. Antibody-based binder is herein understood to refer to molecules or moieties that bind to the target by comprising a structure that is similar to an antibody or part of an antibody, or functions in a similar way as an antibody or part of an antibody. For example, such molecules or moieties may be antibodies, fragment antibody binding (Fab) structures, single-chain variable fragments (scFv), or single-domain antibody (sdAb), also known as nanobodies. In some embodiments, the SSTR2 targeting moiety of the invention is an SSTR2 antagonist or an SSTR2 agonist. SSTR2 agonists typically induce receptor internalization and signal transduction, whereas antagonists mostly block the receptor without inducing internalization and signal transduction. In a preferred embodiment, the SSTR2 targeting moiety is an SSTR2 antagonist that does not induce internalization of SSTR2 upon binding, since receptor internalization hampers effector cell activity.

In another preferred embodiment, the SSTR2 targeting moiety is a somatostatin analogue. Somatostatin analogues are synthetic versions of somatostatin that bind to the same receptors as natural somatostatin.

In preferred embodiments, the SSTR2 targeting moiety is a compound selected from the group consisting of lanreotide, octeotride, pasireotide, and a compound of formula (II):

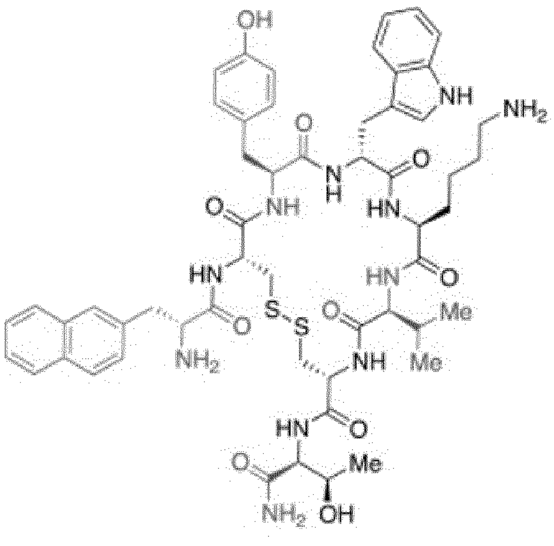

Lanreotide

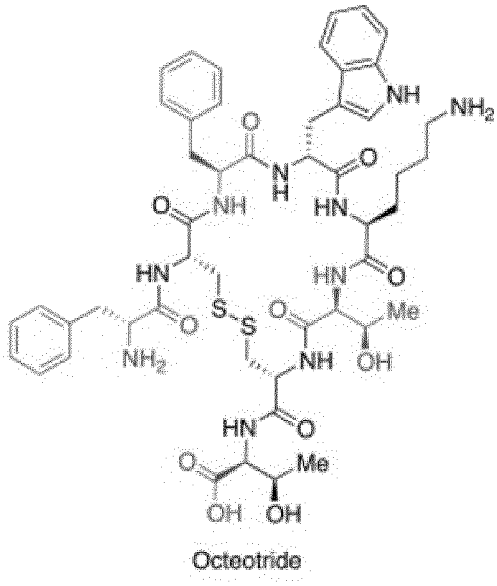

Octeotride

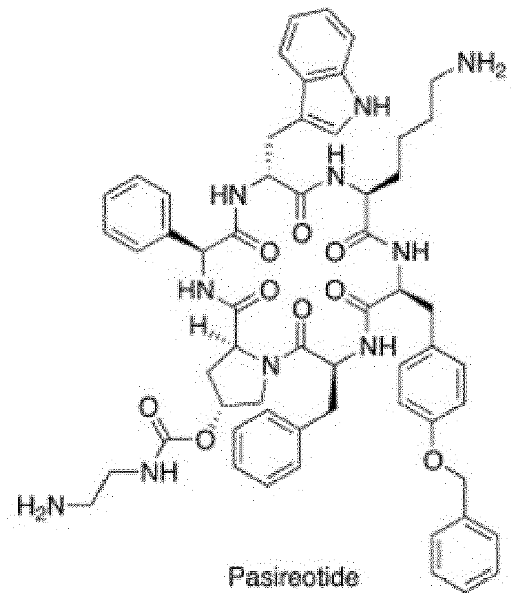

Pasireotide

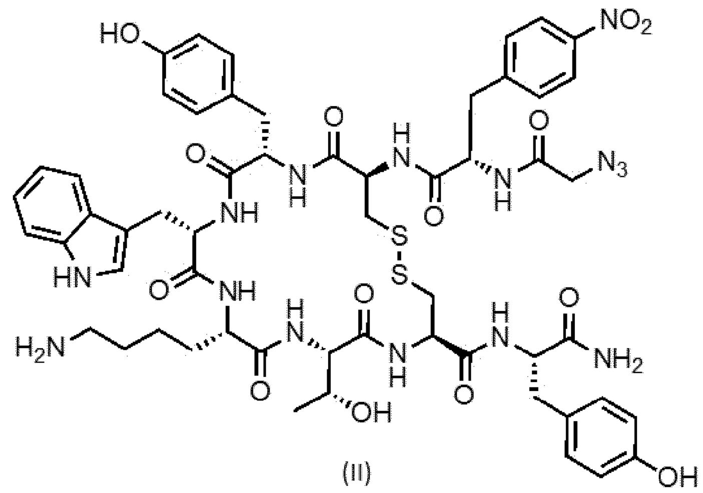

(II)

In a particularly preferred embodiment, the SSTR2 targeting moiety is the compound of formula (II). Compound (II), a SSTR2 antagonist, is a cyclic peptide with high affinity for SSTR2, namely with a $K_D$ below 5 nm based on titration experiments performed using flow cytometry. It does not induce internalization of SSTR2 upon binding.

The adaptor molecule according to the invention comprises a linker moiety connecting the SSTR2 targeting moiety to the tag moiety.

In one embodiment, the linker moiety is a peptide-based compound. Peptide-based compounds have known stabilities and provide a better control over the overall half-life of the adaptor molecule. Peptide-based compounds are typically of low molecular weight, so that adaptor molecules with peptide-based linker moieties have a lower overall molecular weight compared to antibody or protein-based adaptor molecules. This in turn means they show higher penetrance in solid tumors with restricted vasculature, whereas antibody or protein-based adaptor molecules passively diffuse depending on the vasculature of the tumor. Additionally, production of adaptor molecules with peptide-based linkers is more cost-effective compared to antibody or protein-based adaptor molecules. Lastly, integration of peptide-based compounds shortens the half-life to the adaptor molecule in comparison to antibody or protein-based adaptor molecules, which allows for better control over effector-cell activity.

In some embodiments, the linker moiety is hydrophilic and/or soluble in water at physiological pH. The activity of effector-cells may, under certain circumstances, give rise to adverse effects or other unintended consequences in an organism, such as off-target toxicity and cytokine release syndrome (CRS). A hydrophilic profile and solubility under physiological conditions enables control over effector-cell activity by providing the adaptor molecule with a short half-life relative to antibody-based adaptor molecules. This ensures that ceasing administration of the adaptor molecule results in quick effector-cell detachment.

In a preferred embodiment, the linker moiety is a compound of Formula (I):

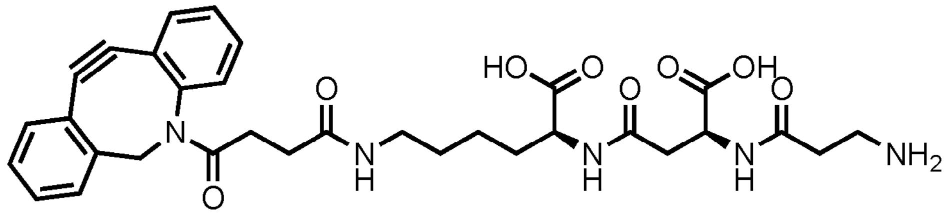

(I)

which shows optimal solubility thus contributing to the overall stability and shelf-life of the adaptor molecule. At the same time, an adaptor molecule comprising this linker moiety shows excellent affinity for SSTR2.

In a further embodiment, the linker moiety is a compound selected from the group comprising:

**A** 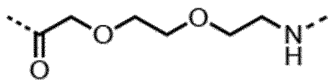

**B** 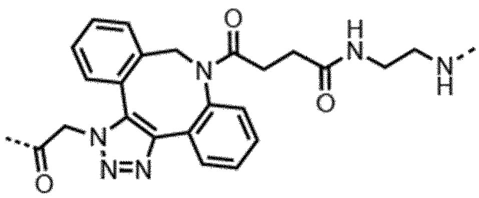

**C** 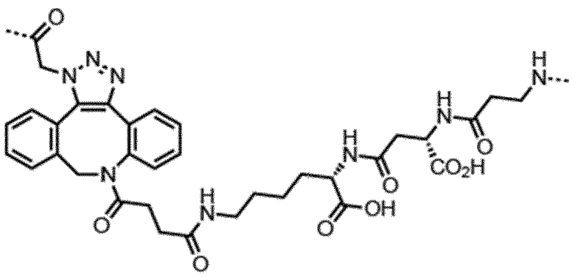

**D** 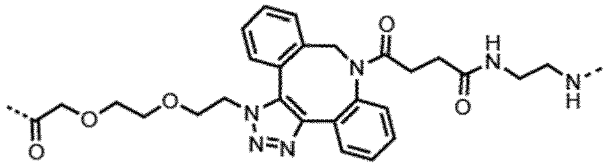

**E** 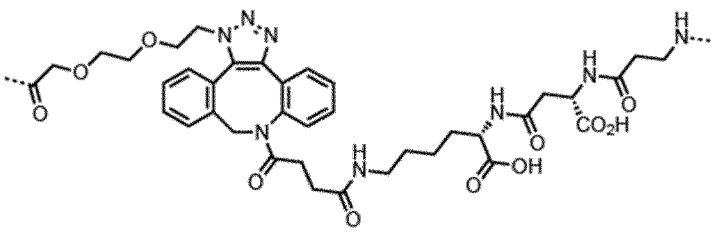

Adaptor molecules using these compounds as linker moiety also show acceptable solubility and affinity for SSTR2.

The adaptor molecule according to the invention further comprises a tag moiety that is recognized either directly by effector cells or by molecules that themselves bind to effector cells.

In one embodiment, the tag moiety is a binding target for an adaptor-CAR T-cell (Ad-CAR T-cell). Binding targets for Ad-CAR T-cells may be antibody constructs or small molecule ligands. By integrating a tag for Ad-CAR T-cells in the adaptor molecule, the interaction between the Ad-CAR T-cells and the tumor cells can thus be regulated.

In another embodiment, the tag moiety is a binding target for another bispecific effector-cell engaging molecule. In other words, the tag moiety is recognized by a molecule that is itself capable of engaging, i.e., binding to an effector-cell. This adds another level of control over the activity of the effector-cells, thus further reducing toxicity and off-target effects. Examples of bispecific effector-cell engaging molecules include T-cell engaging (TCEs) molecules and bispecific T-cell engagers (BiTe). The principle of effector cell engagement is depicted in Fig. 15.

In some embodiments, the tag moiety is a fluorescent molecule. A fluorescently labeled adaptor molecule allows analyzing the performance, efficacy and safety of the adaptor molecule by well-known fluorescence microscopy procedures. For example, a fluorescently labeled adaptor molecule allows validation of the targeting mechanism by showing which tissues or cells the adaptor molecule is binding to, or if the adaptor molecule is accumulating in the absence of SSTR2. Efficacy may be tested by checking for internalization, i.e., verifying if the adaptor molecule has induced internalization of the SSTR2 receptor by the target cancer- or tumor-cell, hampering adaptor CAR T-cell activity.

Furthermore, a fluorescent tag moiety allows detection and identification of target cells during surgical procedures.

In a preferred embodiment, the tag moiety is a binding target for anti-FITC adaptor CAR T-cells. As used herein, the term "anti-FITC adaptor CAR T-cells" refers to CAR T-cells that express receptors targeting fluorescein isothiocyanate (FITC). FITC is a common choice for clinical application, e.g., as a diagnostic dye in ophthalmology or endomicroscopy, where it is injected intravenously without overt safety concerns.

In some embodiments, the tag moiety is selected from the group comprising a biotin moiety, an Fc-binding moiety, a E5B9 10 aa or E7B6 18 aa moiety, a PNE (peptide neo-epitope) 14 aa moiety, a zip-Fv moiety, a SpyTag moiety, an anti-FR (folate receptor) moiety, an anti-SAR (synthetic agonistic receptor) moiety, and a CD19-ECD moiety.

In other embodiments, the tag moiety is capable of being targeted by effector cell such as Fc-binding CAR-T cells, BBIR (biotin-binding immune receptors) CAR-T cells, UniCAR CAR-T cells, anti-PNE scFv CAR-T cells, SUPRA CAR (split, universal, and programmable) CAR-T cells, SpyCatcher CAR-T cells, bispecific antibody-binding immune receptors (BsAb-IR) CAR-T cells, SAR (synthetic agonistic receptors) CAR-T cells, and IMPACT (integrated modules oPtimize adoptive cell therapy) CAR-T cells.

In a further preferred embodiment, the adaptor molecule is a compound of formula (III):

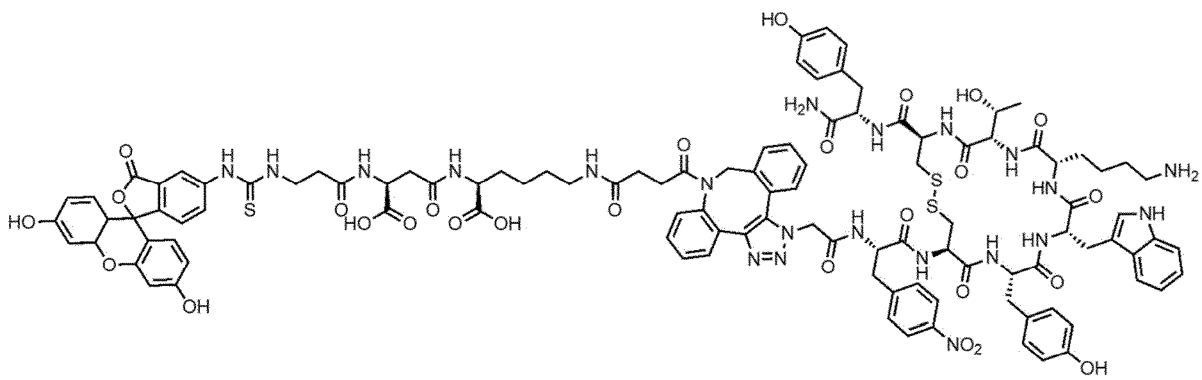

(III)

wherein the SSTR2 targeting moiety is a compound of formula (II), the linker moiety is a compound of formula (I), and the tag moiety is a binding target for anti-FITC adaptor CAR T-cells, respectively.

As demonstrated in the examples below, the compound of formula (III) presents high affinity for SSTR2 without inducing internalization and is therefore capable of targeting SSTR2-overexpressing cells with high selectivity, thereby focusing the activity of effector cells in tumor or cancer tissues. It is particularly suited for use in a targeting approach because of its small size and thus increased access to the target. Acting as a bridge between effector-cells and target cells, the compound of formula (III) offers highly tunable, on-off control over the biocidal activity of the engaged effector-cells. At the same time, fluorescein as tag moiety serves both as effective and safe tag for suited effector-cells and as a tool for analyzing the effects of the adaptor molecule.

In another aspect of the invention, the adaptor molecule according to the invention is for use in the treatment of a tumor, wherein the treatment comprises administration of the adaptor molecule and the administration of immune effector-cells. In one embodiment, the adaptor molecule according to the invention is for use in the treatment of a tumor comprising SSTR2 expressing cells. In another embodiment, the adaptor molecule according to the invention is provided for use in the treatment of a tumor, the tumor being a gastro-entero-pancreatic neuroendocrine tumor, pituitary tumor, paraganglioma, or meningioma.

In another aspect of the invention, the adaptor molecule according to the invention is for use in the treatment of a cancer, the treatment comprising administration of the adaptor molecule and the administration of immune effector-cells. In one embodiment, the adaptor molecule according to the invention is for use in the treatment of a cancer, the cancer being hepatocellular carcinoma or breast cancer.

Preferably, the treatment comprises administration of adaptor-CAR T-cells as immune-effector cells, more preferably administration of anti-FITC adaptor-CAR T-cells.

In some embodiments, wherein the tag moiety is a binding target for a bispecific effector-cell engaging molecule, the treatment additionally comprises administration of the bispecific effector-cell engaging molecule.

In yet another aspect, the invention relates to a method for visualizing a cancer cell, the method comprising targeting the cancer cell with the adaptor molecule according to the invention. The high specificity and accessibility to target tissues of the adaptor molecule allow it to function as a marker for cancer or tumor cells. This in turn makes the adaptor molecule a suitable tool for distinguishing between healthy and malignant cells during diagnostic or surgical procedures, increasing their precision and efficacy.

**Examples**

A new fluorescent-peptide antagonist of SSTR2, termed Octo-Fluo, was generated, validated and tested as adaptor in combination with anti-FITC adaptor-CAR (AdFITC(E2)-CAR) T-cells. Efficient elimination of SSTR2-expressing NET-cells in vitro and in vivo in therapeutic settings was demonstrated in xenogeneic mouse models. Novel generated fluorescent-peptide adaptor molecules antagonist of SSTR2, in combination with anti-FITC adaptor CAR (AdFITC(E2)-CAR) T-cells, were shown to function as an on-off tunable activating bridge between the CAR and SSTR2 expressing target cells.

Overall, in vitro studies confirmed the binding of to Bon1-SSTR2 mCherry-Luc cells without evidence for internalization. AdFITC(E2)-CAR T-cells were activated and efficiently induced Bon1-SSTR2 cell death in vitro, in a manner dependent on adaptor molecule concentration. Similarly, AdFITC(E2)-CAR T-cells in combination with such adaptor molecules efficiently infiltrated the tumor and eliminated Bon1-SSTR2 tumors in immunodeficient mice in therapeutic settings. Both, AdFITC(E2)-CAR T-cell tumor infiltration and biocidal activity were adaptor molecule concentration-dependent, with high doses of adaptor molecule saturating both the CAR and the SSTR2 antigen independently, leading to loss of tumor infiltration and biocidal activity due to loss of bridge formation. It was observed that the biocidal activity of AdFITC(E2)-CAR T-cells co-cultured in vitro with Bon1-SSTR2 mCherry-Luc cells was dependent on the dose of Octo-Fluo bispecific adaptor, indicating that the platform is highly tunable by different amounts of the small molecule bispecific adaptor. In line with this, not only the AdFITC(E2)-CAR T-cell biocidal activity was modulated, also the cytokine release was diminished when the Octo-Fluo molecule dose was at either ends of the optimal dose window. These findings show that the adaptor CAR T-cell systems can be controlled by the presence and amount of bispecific adaptor. Findings demonstrate the potential of using AdFITC(E2)-CAR T-cells with adaptor molecules as a versatile, on-off tunable bispecific adaptor for targeted CAR T-cell immunotherapy against SSTR2-positive NETs.

***Example 1 - Synthesis of compounds of formula (I), (II) and* (III)**

*Resins:*

- Fmoc-Lys(Boc)-Wang resin (loading: 0.4-0.7mmol/g): 47647-1G-F, Sigma Aldrich
- H-Rink amide resin ChemMatrix® resin: 727768-5G, Sigma Aldrich

*Fmoc-protected amino acids:*

- Fmoc-DTyr(tBu)-OH: CAS 118488-18-9, Sigma Aldrich
- Fmoc-LCys(Trt)-OH: CAS 103213-32-7, Sigma Aldrich
- Fmoc-LThr(tBu)-OH: CAS 71989-35-0, Sigma Aldrich

- Fmoc-LLys(Boc)-OH: CAS 71989-26-9, Sigma Aldrich
- Fmoc- DTrp(Boc)-OH: CAS 163619-04-3, Sigma Aldrich
- Fmoc-LTyr(tBu)-OH: CAS 71989-38-3, Sigma Aldrich
- Fmoc- DCys(Trt)-OH: CAS 167015-11-4, Sigma Aldrich
- Fmoc-LPhe(4-$NO_2$)-OH: CAS 95753-55-2, Sigma Aldrich
- Fmoc-LAsp-OtBu, CAS 129460-09-9, Sigma Aldrich
- Fmoc-β-Ala-OH: 35737-10-1, Sigma Aldrich

*Reagents:*

- Azidoacetic acid: CAS 18523-48-3, TCI
- 5-FITC (Fluorescein-Isothiocyanat Isomer I): CAS 3326-32-7, Sigma Aldrich
- 1-(2-Aminoethyl)piperidine: CAS 27578-60-5, ABCR
- TEA: CAS 121-44-8, ABCR
- DIPEA: CAS 7087-68-5, ABCR
- HATU: CAS 148893-10-1, ABCR

*Instruments:*

Analytical HPLC chromatograms and Mass-Spectrometry (MS) spectra were recorded on an Agilent 6100 Series Single Quadrupole MS system combined with an Agilent 1200 Series LC, using an InfinityLab Poroshell 120 EC-C18 Column, 2.7 μm, 4.6 × 50 mm at a flow rate of 0.8 mL/min, 10% ACN in 0.1% aq. FA to 100% ACN in 5 min.

High-Resolution mass spectrometry (HR-MS) were performed on a Q Exactive Mass Spectrometer (Thermo Fisher Scientific). The analyte was injected directly into the MS at a flow rate of 4 μL/min. Spectra were obtained with a resolution of 70000.

Reversed Phase-High Performance Liquid Chromatography (RP-HPLC) was performed on an Agilent 1200 Series RP-HPLC with PDA UV detector, using a Synergi 4μm, Polar-RP 80Å 10 × 150 mm C18 column at a flow rate of 5 mL/min with linear gradients of solvents A and B (A = mQ millipore water with 0.1% TFA, B = ACN with 0.1% TFA).

1.4 Gradient (semi-preparative HPLC): 5% B for 1 min., from 5% to 100% B in 15 min., 100% B for 3 min.

*1. Synthesis of compound of formula (I) (linker)*

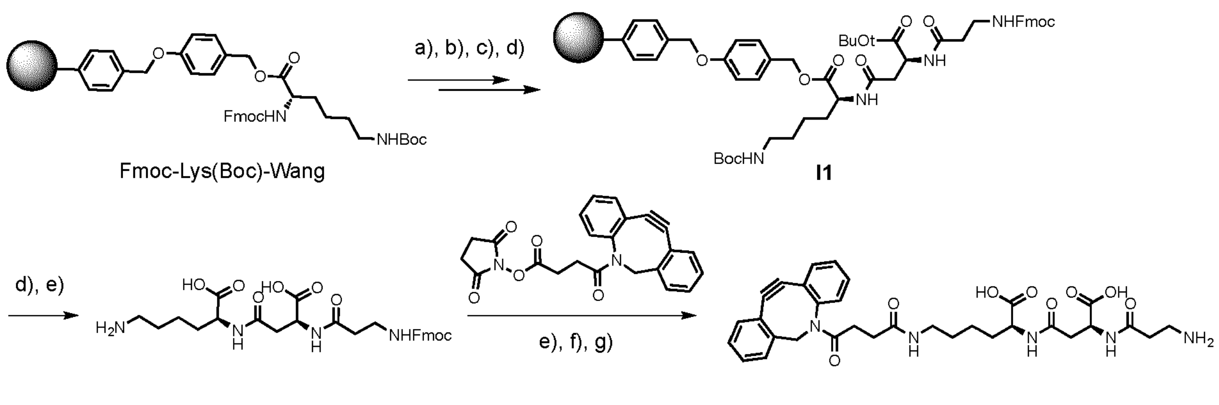


Fmoc-Lys(Boc)-Wang

I1

I2

Compound **1**

Scheme 1: Synthesis of compound of formula (I). Letters correspond to reaction conditions: a) Piperidine 20% in DMF, RT, 3x10'; b) Fmoc-Asp-OtBu (4 eq.), HATU (4 eq.), DIPEA (4 eq.), DMF, RT, 1h; c) Piperidine 20% in DMF, RT, 3x10'; d) Fmoc-βAla (4 eq.), HATU (4 eq.), DIPEA (4 eq.), DMF, RT, 1h; e) TEA (4 eq.), DMSO, 37°C, 1h; f) Piperidine (10 eq.), DMSO, 37°C, 30'; g) Formic Acid (up to pH ~ 3), HPLC purification.

*1.1 Synthesis of intermediate 12*

1.0 g of Fmoc-Lys(Boc)-Wang resin (loading: 0.4-0.7mmol/g) was swollen in 10 mL of dimethylformamide (DMF) for 30 minutes and subsequently, the Fmoc group was deprotected with a solution of 20% piperidine in DMF (3x 10 minutes). For amino acid coupling, Fmoc-Asp-OtBu (Fmoc-L-aspartic acid α-tertbutyl ester) and Fmoc-βAlanine were sequentially coupled to the resin using the following methodology. 4 equivalents of each amino acid were activated with 4 equivalents of HATU and 4 equivalents of DIPEA in 10 mL of DMF. The activated amino acid solution was then added to the resin and reacted for 1 hour at room temperature. After the first coupling step, Fmoc deprotection was carried out by treating the resin with a 20% piperidine solution in DMF (3x 10 minutes). Washing steps were performed after each coupling or deprotection reaction in DMF (6 × 10 mL). The linear peptide I1 was cleaved and deprotected from the resin support by treating the resin with a trifluoroacetic acid (TFA) solution (95% TFA, 2.5% water and 2.5% triisopropyl silane) for 2 hours (3 × 5 mL). The crude Fmoc-protected peptide was dried under reduced pressure and purified by reverse-phase HPLC obtaining the pure peptide I2. The purity of the final peptide I2 was analyzed by analytical HPLC and Mass spectrometry (Fig. 5). MS calculated = 554.23766; MS found [M+1H] = 555.24316. From 1.0 g of resin, 100 mg (0.18 mmol) of pure compound of formula (II) were obtained (total yield = 36%).

*1.2 Synthesis of compound of formula (I)*

10 mg of Fmoc-protected **I2** (18 μmol) and 7 mg of Dibenzocyclooctin-N-hydroxysuccinimidylester (18 μmol) were dissolved in 1 mL of dry DMSO and 10 μL of triethylamine (72 μmol) was added to the solution. The reaction was kept for 1 hour at 37°C. The reaction progress was monitored by LC-MS (MS of product: calculated [M] = 841.3; detected [M+1H] = 842.3, [M-1H] = 840.3; Fig. 6). Upon full conversion of reagents, 20 μL of pure piperidine (0.2 mmol) were added to the reaction and the resulting solution was kept additional 30 mins at 37°C. The reaction progress was monitored by LC-MS (MS product: calculated [M] = 841.3 detected [M+1H] = 842.3, [M-1H] = 840.3; Fig. 7). Upon completion, the reaction was then quenched with formic acid (to pH ~ 3). Pure compound of formula (I) was obtained by reverse-phase HPLC. The purity of the final compound of formula (I) was analyzed by analytical HPLC and High-resolution Mass spectrometry (Fig. 7). MS calculated = 619.26421; MS found [M+1H] = 620.3, [M-1H] = 618.3. 7 mg of pure compound of formula (I) were obtained (11 μmol, total yield = 61%).

*2. Synthesis of Compound of formula (II) (Octo-Azido)*

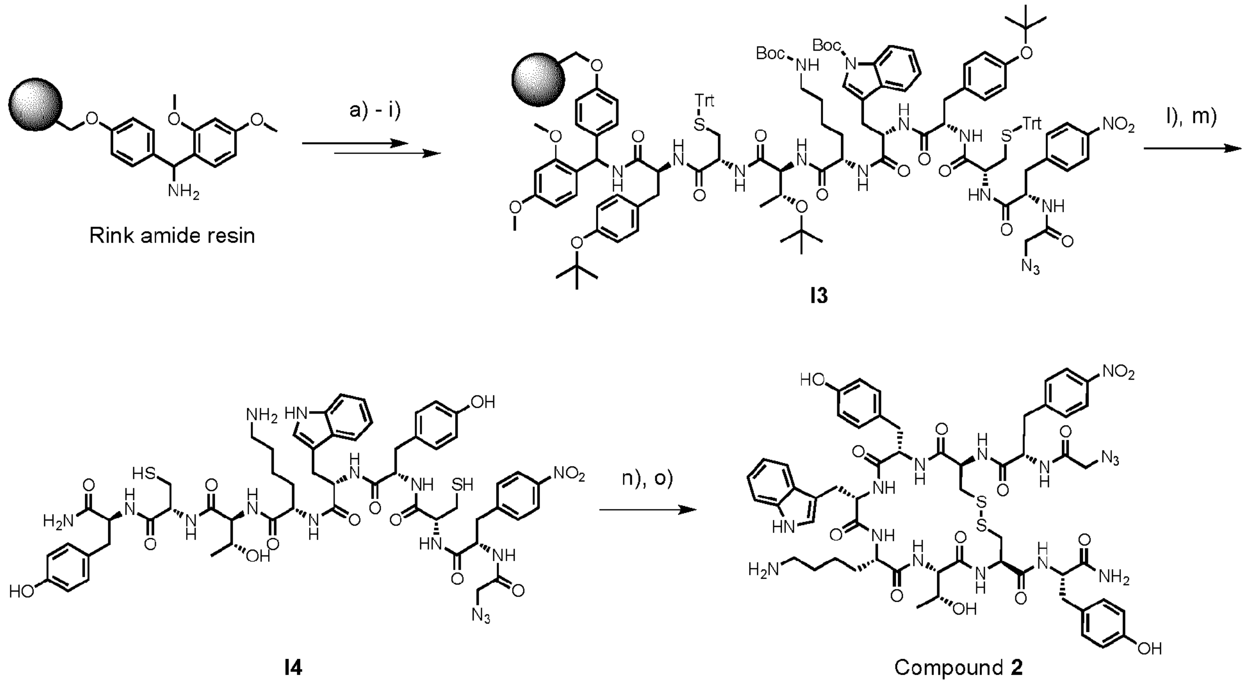


Rink amide resin

I3

I4

Compound **2**

Scheme 2: Synthesis of compound of formula (II). Reaction conditions: Fmoc-protected AA were preactivated with HATU (4 eq.) and DIPEA (4 eq.) in DMF, coupled to the growing peptide (1h at RT) and deprotected after each step of coupling with 20% piperidine in DMF (3x10', RT) in the following order: a) Fmoc-DTyr(tBu)-OH, b) Fmoc-LCys(Trt)-OH, c) Fmoc-LThr(tBu)-OH, d) Fmoc-LLys(Boc)-OH, e) Fmoc- DTrp(Boc)-OH, f) Fmoc-LTyr(tBu)-OH, g) Fmoc-DCys(Trt)-OH, h) Fmoc-LPhe(4-$NO_2$)-OH; i) azidoacetic acid, HATU (4 eq.), DIPEA (4 eq.), DMF, RT, 1h.; l) 92.5% TFA, 2.5% water, 2.5% meta-cresol, and 2.5% thioanisole, 3x2h, RT; m) $Et_2O$, 2h, -20°C; n) DMSO:$H_2O$=1:1, 48h, 37°C. o) HPLC purification.

*2.1 Fmoc-Based Solid-Phase Synthesis* of a *Linear Peptide 13*

*2.1.1 Loading of Rink Amide Resin*

A total of 1.0 g of H-Rink amide resin with a loading capacity of 0.5 mmol/g was weighed and transferred to a reaction vessel (filtered syringe for solid phase synthesis). The resin was then allowed to swell in 10 mL of DMF for 30 minutes to facilitate subsequent reactions.

*2.1.2 Amino Acid Coupling*

Fmoc-based solid-phase synthesis of the linear peptide **I3** was performed by loading the Rink amide resin with the following Fmoc-protected amino acids in the specified order: Fmoc-DTyr(tBu)-OH, Fmoc-LCys(Trt)-OH, Fmoc-LThr(tBu)-OH, Fmoc-LLys(Boc)-OH, Fmoc-DTrp(Boc)-OH, Fmoc-LTyr(tBu)-OH, Fmoc- DCys(Trt)-OH, Fmoc-LPhe(4-$NO_2$)-OH and Azidoacetic acid. 4 equivalents of each Fmoc amino acid and azidoacetic acid were activated using 4 equivalents of HATU and 4 equivalents of DIPEA in 10 mL of DMF. The resulting activated amino acid solution was added to the resin and allowed to react for 1 hour at room temperature.

*2.1.3 Fmoc Deprotection*

After each coupling step, the Fmoc protecting group was removed by treating the resin with a solution of 20% piperidine in DMF. The resin was incubated for 10 minutes at room temperature (3 times).

*2.1.4 Washing*

Following each coupling or deprotection step, the resin was washed six times with 10 mL of DMF in order to remove any unreacted reagent.

*2.1.5 Cleavage and deprotection*

Before cleavage, the resin was swollen in DCM for 3x5 minutes and dried under vacuum. A cleavage solution containing 92.5% TFA, 2.5% water, 2.5% meta-cresol, and 2.5% thioanisole was prepared. The resin was treated for 2 hours with 10 mL of this cleavage solution for three times to ensure complete cleavage and deprotection of the peptide, yielding the crude linear peptide **I4**.

*2.1.6 Precipitation*

The linear peptide product **I4** was precipitated by addition of 5 volumes of diethyl ether to the cleavage solution. The mixture was thoroughly mixed and kept for 2 hours at -20°C. The precipitated peptide was collected by centrifugation, followed by washing with cold (-20°C) diethyl ether. The obtained pellet was dried for 1 hour under vacuum. Intermediate **I4** was analyzed by analytical HPLC and Mass spectrometry (Fig. 8). MS calculated = 1239.5; MS found [M+1H] = 1240.4.

*3. Cyclization of linear peptide I4 to compound of formula (II)*

The dry crude product **I4** was dissolved in DMSO: water = 1:1 reaching a final concentration of 1 mM. The resulting solution was mixed at 37°C for 48 hours. The reaction progress was monitored by LC-MS. Pure compound of formula (II) was obtained by reverse-phase HPLC.

The purity of the final compound of formula (II) was analyzed by analytical HPLC and High-resolution Mass spectrometry (Fig. 9). MS calculated = 1237.44333; MS found [M+1H] = 1238.45874. From 1.0 g of resin, 170 mg (0.14 mmol) of pure compound of formula (II) were obtained (total yield = 27%).

*4. One-pot synthesis of Compound of formula (III) (Octo-Fluo)*

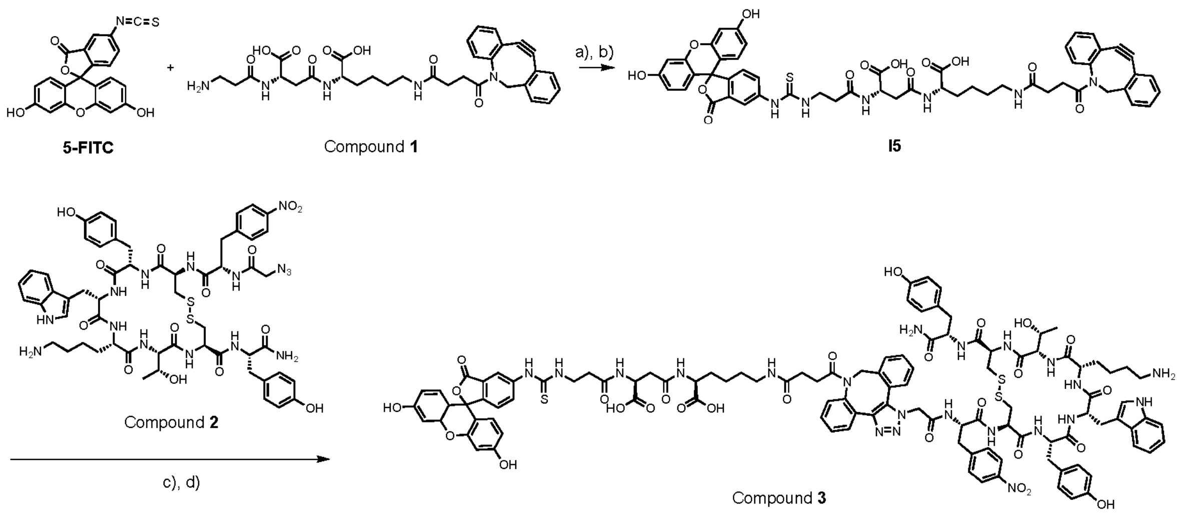


**5-FITC** Compound **1** **I5**

Compound **2**

Compound **3**

<u>Scheme 3</u>: Synthesis of compound of formula (III). Reaction conditions: a) DMSO, TEA (10 eq.), 1h, 37°C; b) 1-(2-Aminoethyl)piperidine (10 eq.), 15', 37°C; c) DMSO, 15', 37°C; d) Formic acid (up to pH ~ 3), HPLC purification.

All the following reactions were carried out in the dark to preserve fluorescein spectrophotometric performance. 7.5 mg of fluorescein isothiocyanate isomer 5 (5-FITC, 19 μmol) and 10 mg of Compound of formula (I) (16 μmol) were

dissolved in 1 mL of dry DMSO and 28 μL triethylamine (0.2 mmol) were added. The resulting solution was mixed for 1 hour at 37 °C. The reaction progress was monitored by LC-MS (Fig. 10).

Upon full conversion of compound of formula (I) to the intermediate **I5**, the excess of 5-FITC was quenched by the addition of 28 μL 1-(2-Aminoethyl)piperidine (0.2 mmol). The reaction was stirred for additional 15 minutes. A solution of compound of formula (II) (Octo-Azido, 20 mg, 16 μmol, 1.0 eq.) in 500 μL of DMSO was then added to I5. The reaction mixed at 37 °C for 15 minutes. The reaction progress was monitored by LC-MS (Fig. 11).

Upon full conversion of **I5** and compound of formula (II) to compound of formula (III), the reaction was quenched with formic acid (to a pH - 3) and pure compound of formula (III) **(Octo-Fluo)** was isolated by reverse-phase HPLC. The purity of the final compound of formula (III) was analyzed by analytical HPLC and High-resolution Mass spectrometry. MS calculated = 2245.74334; MS found [M+1H]/2 = 1123.87817. 25 mg (11 μmol) of pure compound of formula (III) were obtained (total yield = 69%).

***Example 2 - Octo-Fluo production and surface binding validation on Bon1-SSTR2 mCherry-Luc cells.***

The experiment aimed at generating a small molecule targeting SSTR2, which would work as bispecific engager of an anti-FITC Adaptor CAR T-cell platform for the treatment of neuroendocrine tumors (NETs). The SSTR2-binding compound of formula (III), referred to as Octo-Fluo, was obtained through a one-pot procedure, which is described in Fig. 1A and detailed in (Figs. 5 to 7). Initially, compound of formula (I) (linker) and fluorescein isothiocyanate were coupled, followed by quenching of the unreacted FITC. The portable SSTR2 antagonist Octo-azido moiety (Octo-N3) was then added to the dibenzocyclooctyne group (DBCO) via copper-free alkyne-azide cycloaddition (CuAAC) "click" reaction. This strategy enabled the construction of a SSTR2 receptor-binding fragment connected to fluorescein moiety by means of a hydrophilic linker, soluble in water at physiological pH.

Titration assays were performed by flow cytometry to assess the binding affinity of the Octo-Fluo to a Bon1-SSTR2 mCherry-Luc cell line, illustrated as histograms in Fig. 1B and as a sigmoid curve in Fig. 1C. The results confirmed the binding of the Octo-Fluo bispecific adaptor, exhibiting a high affinity towards SSTR2. The immunofluorescence staining of healthy pancreas shown in Fig. 1D was performed using the Octo-Fluo as the primary stain, highlighting the specific binding of the Octo-Fluo to SSTR2 expressed on pancreatic islets.

Furthermore, using the same staining protocol, SSTR2 expressing paraganglioma biopsies were labelled (Fig.1D). In order to validate surface binding, the linker was visualized by confocal microscopy studies (Fig.1E). The Octo-Fluo, indicated in green, demonstrated extracellular membrane coating when bound to SSTR2, as well as no accumulation when SSTR2 was absent, as shown with Bon1-WT cells. The mCherry stain, depicted in red, was included as an internal control for an intracellular signal. Taken together, these findings confirm that the interaction between the Octo-Fluo bispecific adaptor and the Bon1-SSTR2 mCherry-Luc cells results in co-localization and accumulation to the plasma membrane with undetectable internalization in the applied assay.

*1. Synthesis of Octo-Fluo*

All reactions were carried out in the dark to preserve fluorescein spectrophotometric performance. Fluorescein isothiocyanate isomer 5 (5-FITC) and Compound of formula (I) were dissolved DMSO and triethylamine was added. The resulting solution was mixed for 1 hour at 37 °C, upon full conversion of compound of formula (I). The excess of 5-FITC was quenched by the addition of 1-(2-Aminoethyl)piperidine. And subsequently a solution of compound of formula (II) in DMSO was added to the reaction. The resulting mixture was stirred at 37 °C for 15 minutes. Upon full conversion of compound of formula (II) to compound of formula (III), the reaction was quenched with formic acid (to a pH of around 3) and compound of formula (III) (Octo-Fluo) was isolated by reverse-phase HPLC.

*2. Ex vivo terminal analysis and tissue processing*

The homogenized tumor, spleen and blood were treated with Red Blood Cell Lysis Buffer (BioLegend). Once re-suspended in FACS buffer, the all tissue samples were filtered through a 70-μm cell strainer. In order to discriminate the live cells from the dead, the cells were stained with Zombie Aqua Viability Kit (BioLegend) for 30 minutes at 4°C. The tissue samples were subsequently stained using anti-huCD45, anti-muCD45, and anti-huCD3 (Biolegend) for 30 minutes at 4°C. The samples were ultimately strained (30 μm nylon mesh) and analyzed via flow cytometry (BD LSRFortessa™ cell analyser, BD biosciences). The flow cytometry data were analyzed using FlowJo software (Treestar).

*3. Immuno-Fluorescence*

Healthy pancreas and Paraganglioma patient samples were cut (3 μm) and mounted on SuperFrostTM slides (Thermo Fisher Scientific). The tissues were processed by the Department of Pathology, University Hospital Zurich. For

the patient-derived paraganglioma Immuno-Fluorescence staining, slides were subjected to the Octo-Fluo molecule (150nM) as primary stain, followed by rabbit anti-FITC antibody (Catalogue number #4510-7804, BioRad), and donkey anti-rabbit Alexa 488 (Catalogue number #A21206, Invitrogen). In order to visualize the nucleus, the mounting medium was added with DAPI (Abcam) prior to adding the microscope cover glass (Ref. ECN631-1574). Slides were stained with the Ventana Optiview platform (Ventana Medical Systems) according to manufacturer's protocols. The images were taken with an upright epifluorescence microscope (DM5500-B, Leica) at 20x magnification (HC PL FLUOTAR 5x/0.15 Leica objective). Leica LAS-X software was used for microscope control, image acquisition and analyses. All staining intensities were compared to isotype controls.

At terminal analysis of mice, the tumor tissues were cut in half. One half of the tumor was processed by flow cytometry as described below, the other embedded in OCT-medium. The OCT-blocks were then cut into 8 μm slices and mounted on SuperFrost™ Plus slides (Thermo Fisher Scientific). The slides were then permeabilized by placing them in an EasyDip slide staining system (Milian) containing ice cold Acetone. In order to stain the tissues, a hydrophobic square around the sample was drawn using Pap pen (Dako, Catalogue number #S2002). To visualize the $CD3^+$ cells, the slides were stained with the anti-CD3d rabbit monoclonal antibody (Cat. Num. MA5-32462 Invitrogen), followed by rabbit anti-FITC antibody (Catalogue number #4510-7804, BioRad), and donkey anti-rabbit Alexa 488 (Catalogue number #A21206, Invitrogen). In order to visualize the nucleus, the mounting medium was added with DAPI (Abcam) prior to adding the microscope cover glass (Ref. ECN631-1574). The images were taken with an upright epifluorescence microscope (DM5500-B, Leica) at 20x magnification (HC PL FLUOTAR 5x/0.15 Leica objective). Leica LAS-X software was used for microscope control, image acquisition and analyses. All staining intensities were compared to isotype controls.

***Example 3 - Production of AdFITC(E2)-CAR T-cells and in vitro biocidal activity against Bon1-SSTR2 cells in combination with Octo-Fluo***

Given the promising binding properties of Octo-Fluo, anti-FITC Adaptor CAR T-cells were produced, using a previously published anti-FITC single-chain fragment variable (scFv) generated from the sequence of the E2 antibody (Vaughan et al 1996). A lentiviral vector was generated that contains the transgene encoding the AdFITC(E2)-CAR cassette. The second-generation AdFITC(E2)-CAR was linked to RQR8, a marker for gene expression, that may be used for CAR T-cell selection and depletion, via a T2A cleavable linker. The graphical representation depicted in Fig. 1F illustrates the various components of the immunotherapeutic system based on AdFITC(E2)-CAR T-cells and the Octo-Fluo bispecific adaptor, acting as bridging element between effector and SSTR2-expressing target cell. The AdFITC(E2)-CARs were expressed on primary healthy-donor derived T cells (Fig. 12). The cytotoxicity assay consisted of co-incubated AdFITC(E2)-CAR T-cells and Bon1-SSTR2 with Octo-Fluo concentrations varying from 30 fM to 3 μM. Target cell death was measured by flow cytometry at 24, 48 and 72hr (Fig. 2A-C), highlighting an optimal range of concentrations between 0.25 nM and 3 μM where the highest Bon1-SSTR2 depletion occurred. In summary, the biocidal activity of the AdFITC(E2)-CAR T-cells depends on the presence of an adequate concentration of the Octo-Fluo bispecific adaptor, with high activity across a 4-log fold concentration range.

*AdFITC(E2)-CAR T-cell generation and storage*

Healthy donors' buffy coats were acquired from the Zürich blood donation service (Blutspende Zürich, Zürich, Switzerland). PBMCs were enriched by density gradient centrifugation (Ficoll-Paque Plus, GE healthcare). T-cells were then negatively isolated with EasySep™ beads (Human T cell isolation kit, STEMCELL Technologies). Human T-cells were activated in T-cell medium supplemented with CD3/CD28 Dynabeads (ThermoFisher) the day prior to transduction43. For the transduction, Polybrene (Santa Cruz Biotechnology, #134220) was added to a final concentration of 8 μg/ mL and 10 μL virus supernatant was added to the cells and mixed. After centrifugation (1000 g, 90 min, 32°C) the cells were incubated overnight (37°C, 5% CO2). On the following day, the media was exchanged, and the T-cells were incubated overnight (37°C, 5% CO2). The beads were magnetically removed and resuspended in fresh T-cell medium and expanded at $1\times10^6$ cells/mL. The AdFITC(E2)-CAR T-cells were expanded until day 10, when they were cryopreserved and stored at -180°C.

***Example 4 - AdFITC(E2)-CAR T-cell activation is conditional to the presence of both the Octo-Fluo adaptor and tumor-associated antigen-expressing cells***

The next aim was to determine if the adaptor in absence of the tumor-target antigen would activate AdFITC(E2)-CAR T-cells, which would pose a possible safety concern. T-cell and AdFITC(E2)-CAR T-cell activation was ascertained in presence and absence of the Octo-Fluo adaptor with or without SSTR2-expressing target cells by respective co-incubation assays. Fig.2D illustrates the gating strategy and data acquired by flow cytometry after 48 hours of exposure to each condition. Both AdFITC(E2)-CAR T-cells and control T-cells (Fig. 13) were not activated in the presence of Octo-Fluo alone, as indicated by low or absent expression of CD69/CD25 and PD1. In stark contrast, when the

AdFITC(E2)-CAR T-cells were exposed to Bon1-SSTR2 cells and Octo-Fluo (Fig. 2D), CD69/CD25 and PD1 expression increased to 58%/89.5% and 77%, respectively. Meanwhile, when AdFITC(E2)-CAR T-cells were exposed to SSTR2 negative tumor cells (Bon1-WT) and Octo-Fluo, CD69/CD25 and PD1 expression increased to 48/37% and 15.4%, respectively. In order to test if phenotypic T-cell activation correlated with cytokine release, IL-2 and IFNy was measured in supernatants of co-cultures in each condition (Fig. 2E). The results demonstrate strong cytokine release only in the presence of all elements, AdFITC(E2)-CAR T-cells, Octo-Fluo and Bon1-SSTR2. Thus, these data indicate that binding of Octo-Fluo to AdFITC(E2)-CAR T-cells without crosslinking to SSTR2-expressing target cells will likely not cause a safety limitation of the approach.

### *Example 5 - Tumor eradication by AdFITC(E2)-CAR T-cells in vivo*

To determine the efficacy of AdFITC(E2)-CAR T-cells in combination with Octo-Fluo in vivo, $8\times10^6$ Bon1-SSTR2 mCherry-Luc tumor cells were subcutaneously (s.c.) implanted in the flank of NSG mice. After 14 days, once the tumors reached an average size of 80mm3, mice were treated by intravenous (i.v.) administration of $10^7$ AdFITC(E2)-CAR T-cells, with or without concomitant i.v. injection of Octo-Fluo (2.5 or 0.5 nmol every 12h). The experimental setup is shown in Fig. 3A. In mice receiving PBS and only AdFITC(E2)-CAR T-cells, the tumors grew in similar kinetic and size, whereas tumors were eradicated by twice daily additional application of Octo-Fluo (Fig. 3B-D). The quantification of the total flux illustrated in Fig. 3C underlines the slower tumor eradication kinetics of the 2.5 nmol Octo-Fluo group compared to the reduced dose group of 0.5 nmol. The tumor volume and body weight change shown in Fig. 3D, highlight a marked improvement of health in the groups that received 0.5 and 2.5 nmol of Octo-Fluo, whereas the control groups showed a decrease in body weight due to the tumor outgrowth. Overall, the results in Fig. 3 underline the potent anti-tumor response when injected with both doses as well as the marked improvement of health (as determined by weight) in comparison to the negative controls PBS and AdFITC(E2)-CAR T-cells without adaptor where similar rates of tumor growth were reported.

*In vivo therapeutic studies*

All procedures involving experimental animals were performed according to protocols approved by the Cantonal Veterinary Office Zurich (006/2021 and 134/2022). On day 0, NSG mice (NOD.Cg-Prkdcscid Il2rgtm1Wjl/SzJ) were transplanted s.c. with $8\times10^6$ Bon1-SSTR2 mCherry-Luc cells. Once the tumor volume reached about $80mm^3$, $10^7$ AdFITC(E2)-CAR T-cells were injected i.v. followed immediately by an injection i.v. of Octo-Fluo at the indicated amount. Tumor burden was evaluated on a weekly basis by BLI and daily caliper measurements. The Octo-Fluo i.v. injections were repeated every 12hr until the termination of the experiment.

### *Example* **6** *- Excess administration of Octo-Fluo in vivo inhibits AdFITC(E2)-CAR T-cell tumor infiltration and function*

In order to further assess the Octo-Fluo dose-dependent effect, the experiment was repeated with the working doses of 0.5 and 2.5 nmol and a higher dose of 12.5 nmol added to test if this would quench the activity due to saturating both the SSTR2 and the AdFITC(E2)-CARs. Fig. 4A summarizes the experimental setup. Briefly, $8\times10^6$ Bon1-SSTR2 mCherry-Luc tumor cells were engrafted s.c. in the flank of NSG mice. After 14 days, mice were administered i.v. $10^7$ AdFITC(E2)-CAR T-cells. For this experiment, three increasing doses of Octo-Fluo were i.v.-injected every 12h: 0.5, 2.5, and 12.5 nmol.

As shown Fig. 14, tumor volume measurements indicate that twice daily injection of 12.5 nmol of Octo-Fluo inhibited the anti-tumor response by saturating both the AdFITC(E2)-CARs as well as SSTR2 present on the tumor cell. This resulted in tumor outgrowth, while 2.5 and 0.5 nmol both exhibited tumor responses. In order to further understand the mechanism behind these different outcomes, the distribution of the AdFITC(E2)-CAR T-cells was analyzed in blood, spleen and tumors, as shown in Fig.4B. The findings illustrate that regular injections of 12.5 nmol Octo-Fluo resulted in the absence of AdFITC(E2)-CAR T-cells in the tumor microenvironment, which instead remained present in the blood and spleen, displaying similar patterns as the AdFITC(E2)-CAR T-cells without Octo-Fluo injection. Meanwhile, when mice were treated with 2.5 nmol of Octo-Fluo, there was a marked increase in AdFITC(E2)-CAR T-cells in the tumor mass, while only a low population density was observed in the blood and spleen. Further, the AdFITC(E2)-CAR T-cell distribution in mice treated with 0.5 nmol Octo-Fluo indicated that once the tumor was eradicated, the AdFITC(E2)-CAR T-cells remained mainly present in the blood and spleen. Fig. 4B depicts flow cytometry plots and immuno-fluorescence staining of tumors. Together, these data suggest that the dose of the bispecific adaptor Octo-Fluo impacts on the ability of the AdFITC(E2)-CAR T-cell to infiltrate and ultimately support a tumor response.

Being a small molecule conjugated with a single fluorescein, Octo-Fluo is characterized by a monovalent engagement of the AdFITC(E2)-CAR molecule. This interaction is similar to the engagement bispecific antibodies achieve as they engage the TCR complex monovalently, preventing systemic activation of effector-cells in the absence of the target

cells. Previous studies suggested that CAR T-cell activation relies on CAR dimerization, and that monomeric interactions do not elicit an activation. Here it was shown that the exposure of the AdFITC(E2)-CAR T-cell to the Octo-Fluo adaptor without the presence of target antigen does not induce dimerization, hence activation was not observed by CD69 and CD25 measurements. Only when both elements are exposed to an SSTR2-expressing cell line, the tumor cells act as a solid support for multiple interactions, leading to dimerization and ultimately AdFITC(E2)-CAR T-cell activation.

The in vivo experiments investigating the impact of the dose at a fixed injection schedule of Octo-Fluo highlighted the importance with respect to both the activity as well as the biodistribution of the AdFITC(E2)-CAR T-cells. These findings support previously published studies where it was shown that increasing 5-fold the optimal dose led to tumor persistence. In the present experiment, it was observed that 25-fold the optimal dose resulted in comparable tumor growth to the negative control groups that received PBS or AdFITC(E2)-CAR T-cells without adaptor. Not only did the dose quench the AdFITC(E2)-CAR and SSTR2 binding sites, but it also resulted in poor AdFITC(E2)-CAR T-cell tumor infiltration.

***Example* 7 - *Supplementary materials and methods***

1. *Cell lines*

Bon1-SSTR2/WT (CVCL_3985, obtained from Japanese Collection of Research Bioresources) mCherry-Luc NET cell lines were kept in DMEM F12 medium supplemented with 10% FBS (Gibco, #10270106) and 1% Pen-Strep. HEK293T cells (ATCC® CRL-3216™) were kept in DMEM media (Gibco), supplemented with 10% FBS (Gibco, #10270106), 2mM Glutamine and 1% Pen-Strep. T-cells were expanded in Advanced RPMI (Gibco) supplemented with 10% FBS (Gibco, #10270106), Glutamax, 1% Pen-Strep and 100 UI of human IL-2/ml (PeproTech).

*2. AdFITC(E2)-CAR T-cell viral production*

The EF1-AdFITC(E2)-CAR-T2A-RQR8 was cloned using restriction sites NheI (NEB) and XhoI (NEB) to insert the anti-Fluorescein scFv E2. The sPAX2 packaging and pCAG-VSVG envelope plasmids were both kindly provided by Prof. Dr. Patrick Salmon (University of Geneva, Switzerland)[20]. The RQR8 sequence was kindly provided by Prof. Dr. Brian Philip and Prof. Dr. Martin Pule (University College London, United Kingdom) and was inserted as a marker for expression[10].

HEK293T-cells were seeded at a density of $5\times10^6$ in 15 mL of complete DMEM media (as previously described) in a T75 cell culture flask. The transfection mixture was added to the HEK293T cells and incubated at 37°C and 5%$CO_2$. The complete DMEM media was replaced after 4-6 hours with fresh medium and the supernatant was harvested the following day. To concentrate the virus the supernatant was transferred to a 50 mL Falcon tube and spun (400g, 5 min, RT) and filtered using 0.22 μm filter (TPP, Switzerland) to remove cells and debris. To concentrate the virus, PEG-it (System Biosciences, CA, USA) was added according to the manufacturer's instructions and samples were incubated overnight at 4°C. The next morning, samples were centrifuged (1500g, 30 min, 4°C). After aspirating the supernatant, the pellet was centrifuged again (1500g, 30 min, 4°C) to remove residual supernatant. The pellet was resuspended in 1/200 of the original plating volume and stored at -80°C until further use.

*3. Generation of Bon 1-SSTR2 mCherry-Luc cell lines*

A PCR-amplified product spanning the entire coding sequence of hSSTR2 was ligated into TOPO cloning vector and subcloned into the pcDNA™3.1(+) expression vector (both vectors: Thermo Fisher Scientific, Waltham, Massachusetts, US). The insert was verified by full-length sequencing. Stable transfection of BON1 cells with the pCR3.1/hSSTR2 insert was performed with Lipofectamine (Thermo Fisher Scientific) according to the manufacturer's instructions. Subsequently, cells were cultured in selection medium containing 1mg/ml geneticin for 4 weeks before single pCR3.1/hSSTR2 clones were isolated. All clones were further expanded in selection medium containing geneticin (1mg/ml). For further experiments, one cell clone was selected, based on maximal hSSTR2 protein expression as determined by Western Blot analysis. The mCherry-Luciferase vector was cloned by inserting the mCherry cassette in the lentiviral dual reporter vector CMV-GFP-T2A-Luciferase plasmid (System Biosciences), by digestion with BamHI and AgeI. Once the sequence was confirmed, lentiviral vectors were produced using the same sPAX2 and pCAG-VSVG as previously mentioned. The Bon1-SSTR2 cells were transduced using Polybrene (Santa Cruz Biotechnology, #134220), added to a final concentration of 8 μg/ mL, and 10 μL virus supernatant was added to the cells and mixed. After centrifugation (1000 g, 90 min, 32°C) the cells were incubated overnight (37°C, 5% $CO_2$). On the following day, the media was exchanged, and the transduced cell lines were expanded for 3 passages. Once reached $10^6$ Bon1-SSTR2 mCherry-Luc bulk cells was reached, single-cell sorting was ran using the FACSAria III 4L flow cytometer (Becton Dickinson) to isolate the best expressers of mCherry-Luciferase and incubated at (37°C, 5% $CO_2$). The selected cells were then cultured and validated for mCherry expression and cryopreserved in freezing media (90% FBS supplemented with 10% DMSO)

and stored in liquid nitrogen at -180°C.

*4. Flow cytometry*

Extracellular antigens of interest were stained with fluorescently-labelled antibodies (see Table 1 below). Data was acquired using the BD LSRFortessa™ II flow cytometer (Becton Dickinson). FlowJo (v10.0.7, FlowJo) was used for data analysis and presentation. The data were compensated and exported with FlowJo software (version 10, TreeStar Inc.).

*5. T-cell Isolation and storage*

Healthy donor buffy coats were acquired from the Zurich blood donation service (Blutspende Zurich, Switzerland). Peripheral blood mononucleated cells (PBMCs) were enriched by density gradient centrifugation (Ficoll-Paque Plus, GE healthcare). The T-cells were then negatively isolated using the EasySepTM beads (human T-cell isolation kit, STEMCELL Technologies). Purified T-cells were cryo-preserved in freezing media (90% FBS supplemented with 10% DMSO) and stored in liquid nitrogen at -180°C.

*6. Octo-Fluo binding to cells*

150'000 Bon1-SSTR2/WT mCherry-Luc cells were resuspended in 100 μL of 100 nM Octo-Fluo, and incubated for 1 hour at 4°C. Subsequently, the cells were centrifuged (400 g, 5 min, 4°C), and the supernatant was removed. Finally, the pellet was resuspended in 200 μL of FACS buffer (2% FBS and 2 mM EDTA) and analysed (BD LSRFortessa™ cell analyser, BD biosciences). The flow cytometry data were analysed using FlowJo software (Treestar).

*7. Confocal microscopy*

Bon1-SSTR2 and WT mCherry-Luc cells were seeded into 8-well coverslip chamber plates (Ibidi) at a density of $5\times10^4$ cells per well in complete DMEM-F12 medium (1 mL, Invitrogen) and allowed to grow for 24h under standard culture conditions. Hoechst 33342 nuclear dye (Invitrogen) was used to stain nuclear structures. The culture medium was replaced with fresh medium containing Octo-Fluo (100 nM). Randomly selected colonies were captured 1h after incubation on an SP8 confocal microscope equipped with an AOBS device (Leica Microsystems).

*8. In vitro cytotoxicity assays*

On day 0, Bon1-SSTR2/WT mCherry-Luc were seeded at a density of 30'000 cells per well, in a 96-well plate and incubated overnight (37°C, 5% $CO_2$). AdFITC(E2)-CAR T-cells were added at a 1:1 effector to target ratio to the seeded Bon1-SSTR2/WT mCherry-Luc in 200 μL Advanced RPMI (Gibco) containing different concentrations of Octo-Fluo. The plates were then incubated killing rate was assessed at three-time points, 24, 48 and 72 hours via flow cytometry. The supernatant was transferred to a round bottom 96 well plate for later cytokine analysis. After washing the wells with 100 μL of PBS, the PBS was also collected. Then, 50 μL Trypsin (Gibco) was added to each well and incubated for 5 minutes at 37°C to detach the target cells. The detached cells were added to the corresponding well of the round bottom 96 well plate. The plate was spun down (400 g, 5 min, RT), flicked to remove the supernatant and the pellets were resuspended in 100 μL FACS buffer containing the Zombie Violet stain (Biolegend). After 30 min of incubation with the FACS buffer at 4°C in the dark, the cells were spun down (400 g, 5 min, 4°C). The plates were subsequently stained using anti-huCD3 (Biolegend) for 30 minutes at 4°C. The assay was ultimately strained (30 μm nylon mesh) and analyzed via flow cytometry (BD LSRFortessa™ cell analyser, BD biosciences). The flow cytometry data were processed using FlowJo software (Treestar). Percentage of specific lysis was calculated as shown below:

$$\textit{Specific Lysis}\ (\%) = \frac{(1\text{-}\textit{number of live target cells})}{\textit{number of live target cells in negative controls}}\ \text{x}100$$

*9. Cytokine release assays*

The cytokine expression was measured using the Enzyme-linked immunosorbent assays (ELISA) MAX Deluxe set human IL-2 and IFNy (Biolegend). Briefly, 100 μL of serum was taken from the cytotoxicity assays at 24hr, 48hr and 72hr and placed in a 96-well plate and stored at -20°C. For the analysis, the serum was thawed and ran the ELISA following

the standard protocol suggested by the manufacturers. For the serum dilution, 1:100 and 1:200 were used for the IL-2 and IFNγ respectively.

| Target | Clone | Fluorochrome | Supplier |
|---|---|---|---|
| hCD3 | UCHT1 | Brilliant Violet 711 ™ | Biolegend |
| hCD4 | OKT4 | APC | Biolegend |
| hCD8 | SK1 | PE-Cy7 | Biolegend |
| hCD34 | QBEND-10 | PE | Thermo Fischer |
| hCD45 | HI30 | eFluor™ 450 | Ebiosciences |
| mCD45 | 30-F11 | PE-Cy7 or Alexa Fluor™ 700 | Ebiosciences |

Supplementary Table 1: List of commercial antibodies used for *in vitro* and *ex vivo* analysis.

## Claims

1. An adaptor molecule for targeting a cancer cell, comprising

   a somatostatin receptor 2 (SSTR2) targeting moiety,
   a linker moiety, and
   a tag moiety,
   wherein the SSTR2 targeting moiety is connected to the tag moiety by means of the linker moiety.

2. The adaptor molecule of claim 1, wherein the adaptor molecule is a small molecule with a molecular weight of ≤ 1000 Da.

3. The adaptor molecule according to claim 1 or 2, wherein the SSTR2 targeting moiety is an SSTR2 antagonist or an SSTR2 agonist, preferably a somatostatin analogue.

4. The adaptor molecule according to claims 1 to 3, wherein the SSTR2 targeting moiety is a compound selected from the group consisting of:

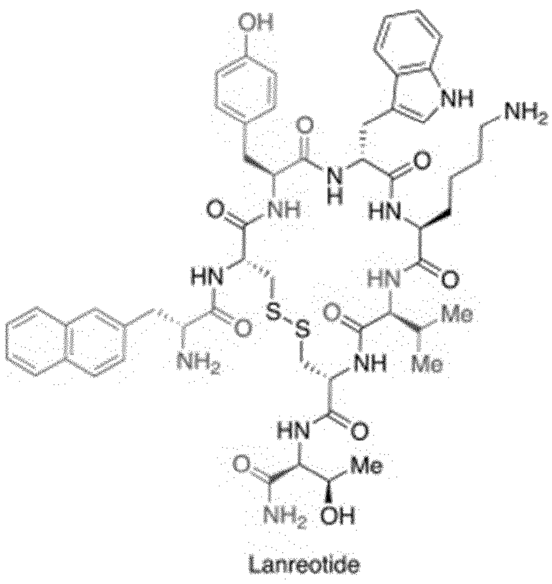

Lanreotide

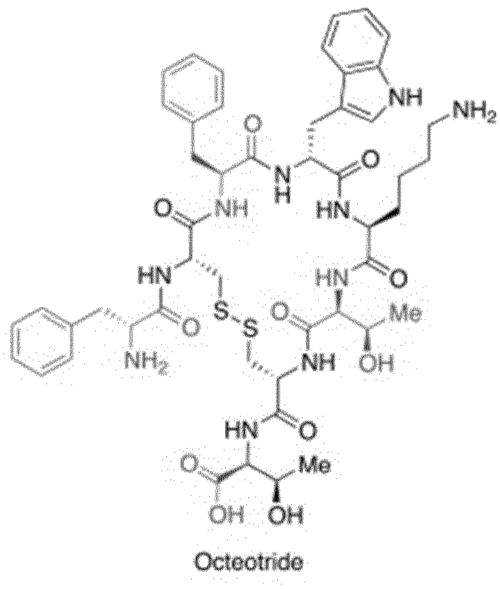

Octeotride

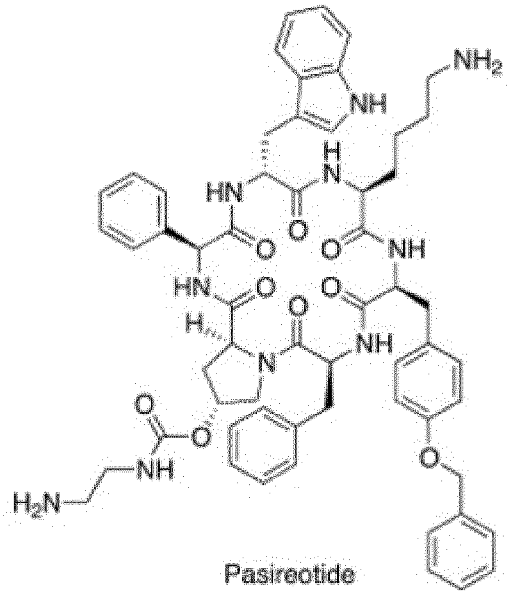

Pasireotide

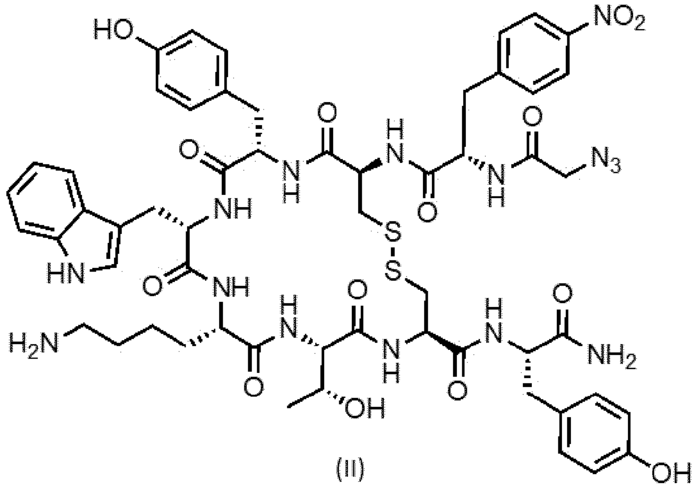

(II)

5. The adaptor molecule according to any of claims 1 to 4, wherein the linker moiety is a peptide-based compound.

6. The adaptor molecule according to any of claims 1 to 5, wherein the linker moiety is hydrophilic and/or soluble in water at physiological pH.

7. The adaptor molecule according to any of claims 1 to 6, wherein the linker moiety is a compound of Formula (I):

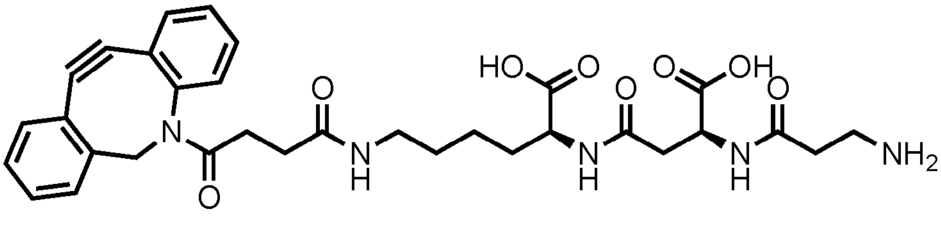

(I)

8. The adaptor molecule according to any of claims 1 to 7, wherein the tag moiety is a binding target for an adaptor-CAR T-cell or a bispecific effector-cell engaging molecule.

**9.** The adaptor molecule according to any of claims 1 to 8, wherein the tag moiety is a fluorescent molecule and/or wherein the tag moiety is a binding target for anti-FITC adaptor CAR T-cells.

**10.** The adaptor molecule according to any of claims 1 to 9, wherein the adaptor molecule is a compound of Formula (III):

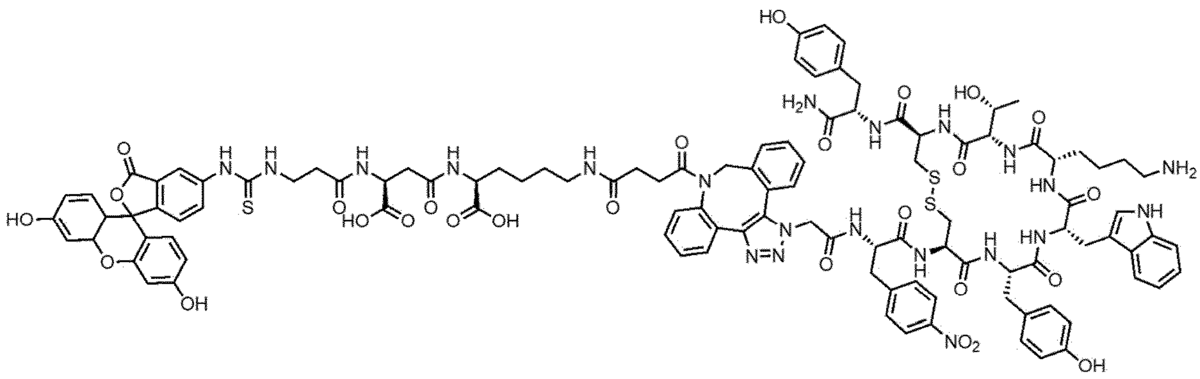

(III)

**11.** The adaptor molecule according to any of claims 1 to 10 for use in the treatment of a tumor, wherein the treatment comprises administration of the adaptor molecule and the administration of immune effector cells, optionally also comprising the administration of bispecific effector-cell engaging molecules and/or T-cell engaging molecules (TCEs).

**12.** The adaptor molecule for use according to claim 11, wherein the tumor comprises SSTR2 expressing cells.

**13.** The adaptor molecule according to any of claims 1 to 10 for use in the treatment of cancer, wherein the treatment comprises administration of the adaptor molecule and the administration of immune effector cells.

**14.** The adaptor molecule for use according to any of claims 11 to 13, wherein the immune effector cells are anti-FITC adaptor-CAR T-cells.

**15.** A method for visualizing a cancer cell, the method comprising targeting the cancer cell with the adaptor molecule according to any of claims 1 to 10.

Figure 1

**A**

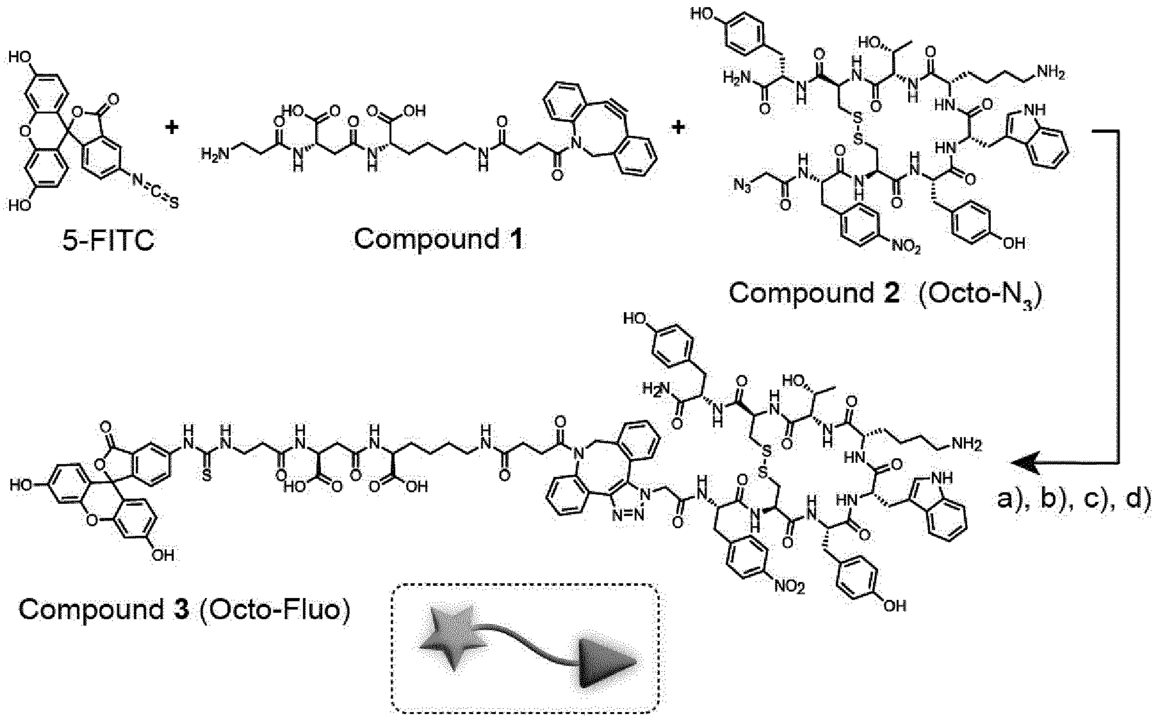


5-FITC

Compound **1**

Compound **2** (Octo-$N_3$)

Compound **3** (Octo-Fluo)

**B**

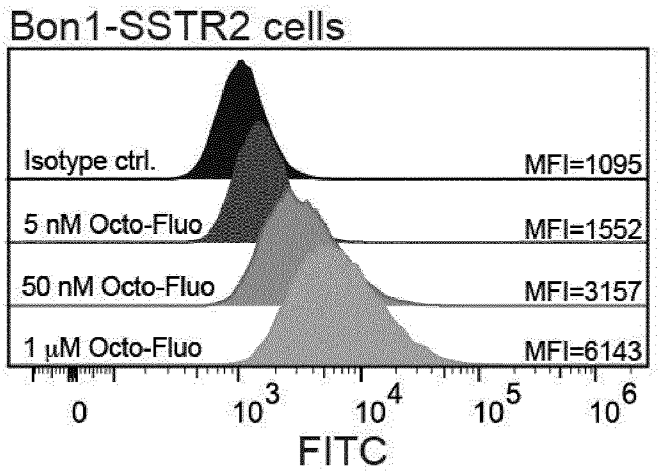

Figure 1

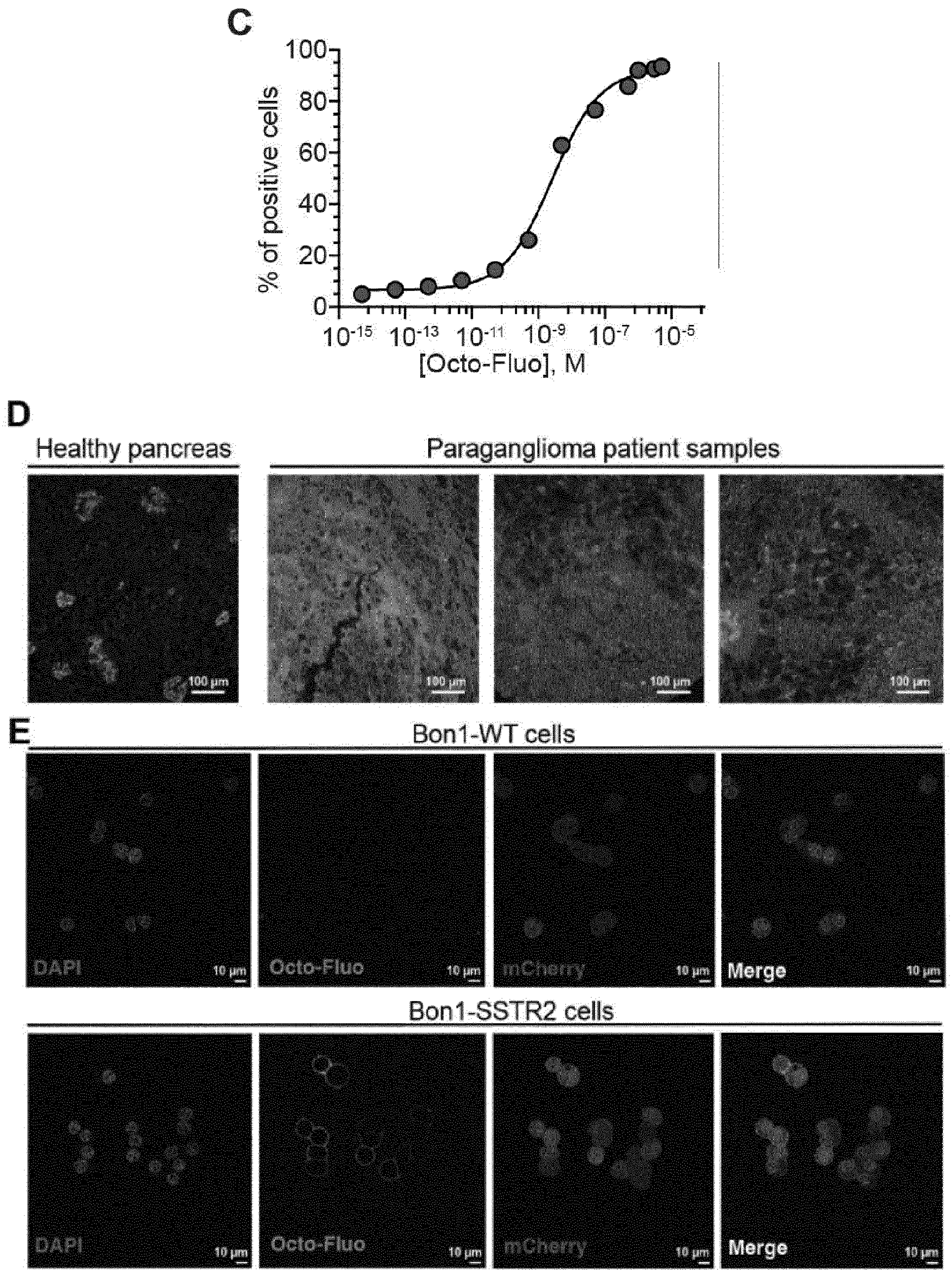
C
100
80
60
40
20
0
% of positive cells
10-15 10-13 10-11 10-9 10-7 10-5
[Octo-Fluo], M
D
Healthy pancreas
Paraganglioma patient samples
100 µm
E
Bon1-WT cells
DAPI
Octo-Fluo
mCherry
Merge
10 µm
Bon1-SSTR2 cells

Figure 1

F

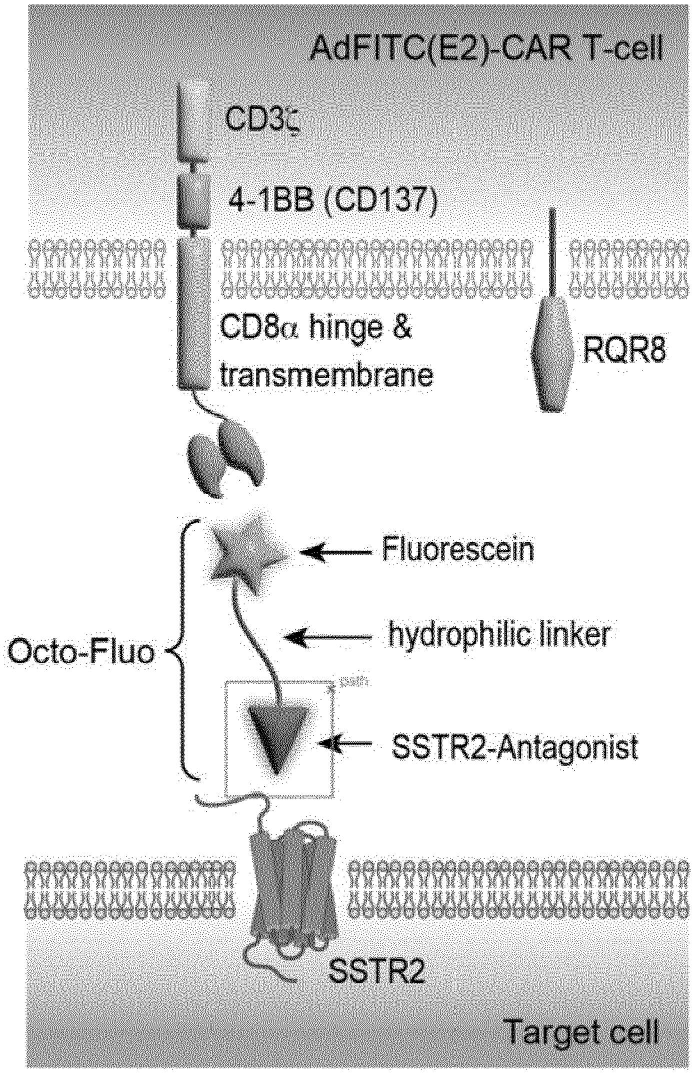
AdFITC(E2)-CAR T-cell
CD3ζ
4-1BB (CD137)
CD8α hinge &
transmembrane
RQR8
Fluorescein
hydrophilic linker
Octo-Fluo
SSTR2-Antagonist
SSTR2
Target cell

Figure 2 A

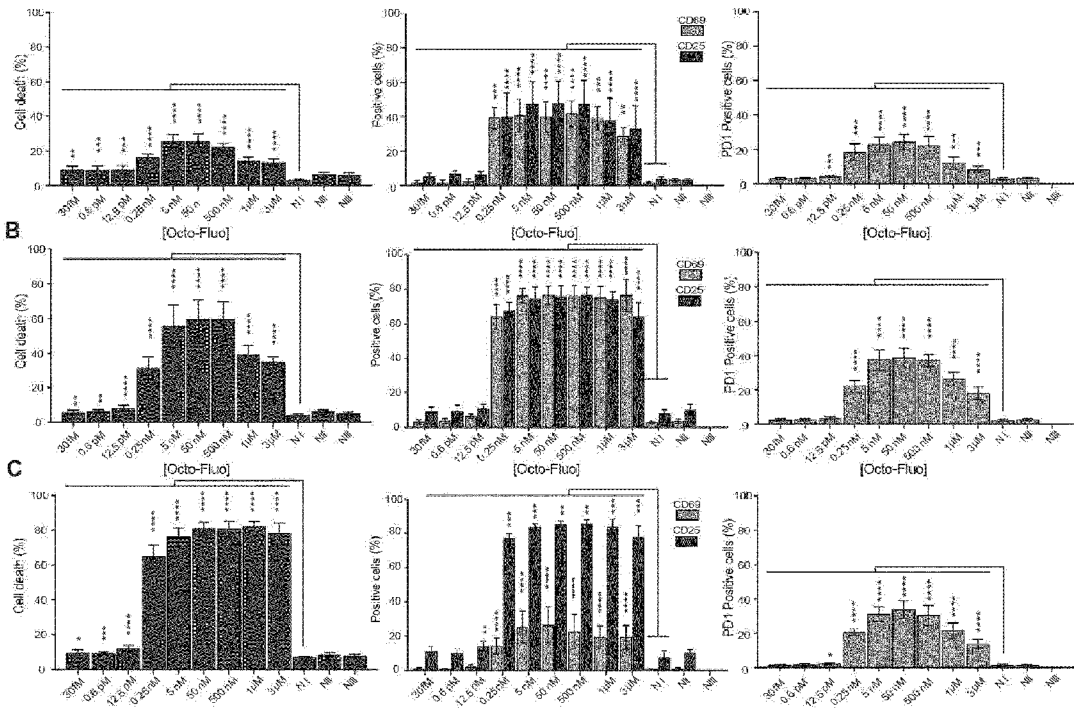
B
C
Cell death (%)
Positive cells (%)
PD1 Positive cells (%)
CD69
CD25
[Octo-Fluo]
30fM
0.6 pM
12.5 pM
0.25nM
5 nM
50 nM
500 nM
1uM
3uM
NI
NII
NIII

Figure 2

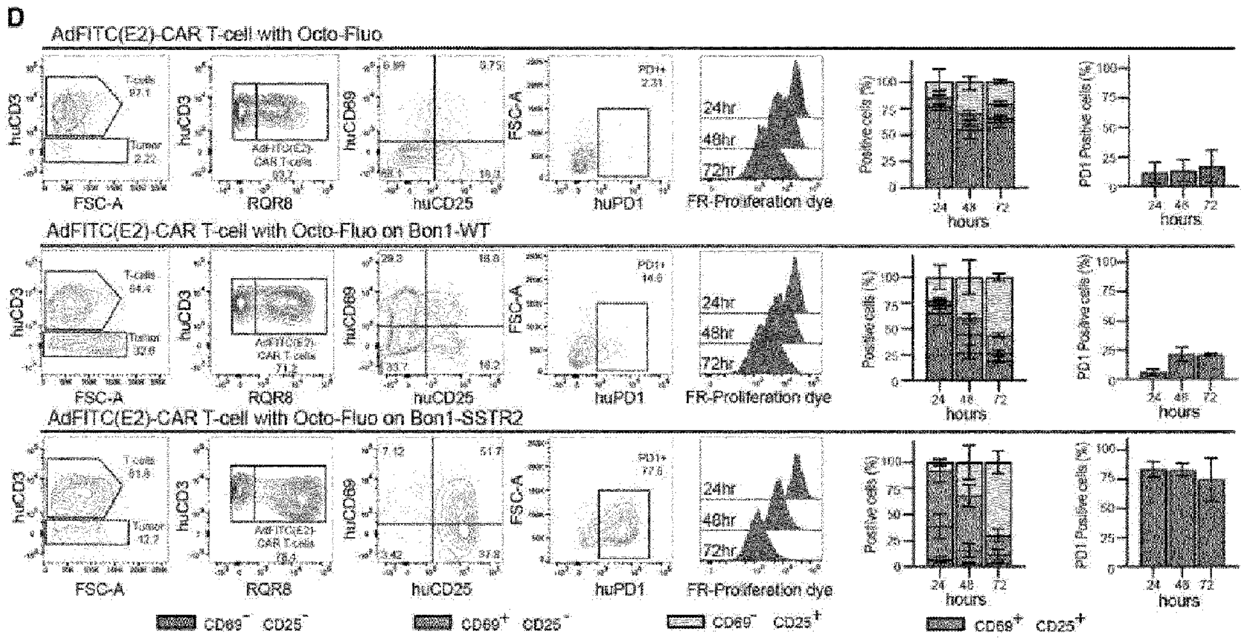
D
AdFITC(E2)-CAR T-cell with Octo-Fluo
AdFITC(E2)-CAR T-cell with Octo-Fluo on Bon1-WT
AdFITC(E2)-CAR T-cell with Octo-Fluo on Bon1-SSTR2
huCD3
FSC-A
RQR8
huCD69
huCD25
FSC-A
huPD1
24hr
48hr
72hr
FR-Proliferation dye
Positive cells (%)
PD1 Positive cells (%)
hours
CD69⁻ CD25⁻
CD69⁺ CD25⁻
CD69⁻ CD25⁺
CD69⁺ CD25⁺

Figure 2

E

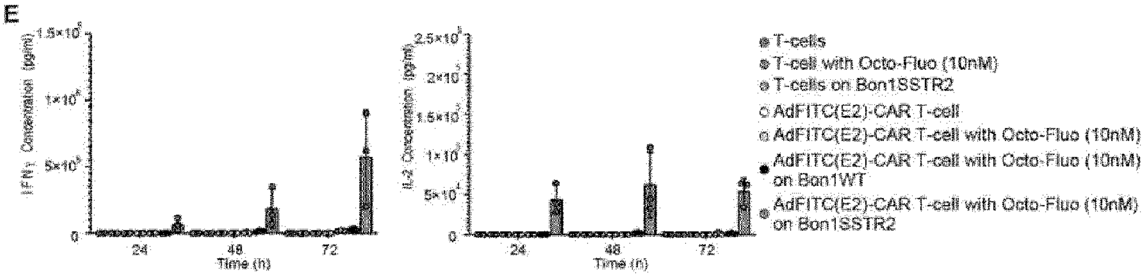
T-cells
T-cell with Octo-Fluo (10nM)
T-cells on Bon1SSTR2
AdFITC(E2)-CAR T-cell
AdFITC(E2)-CAR T-cell with Octo-Fluo (10nM)
AdFITC(E2)-CAR T-cell with Octo-Fluo (10nM) on Bon1WT
AdFITC(E2)-CAR T-cell with Octo-Fluo (10nM) on Bon1SSTR2
Time (h)
24
48
72

Figure 3

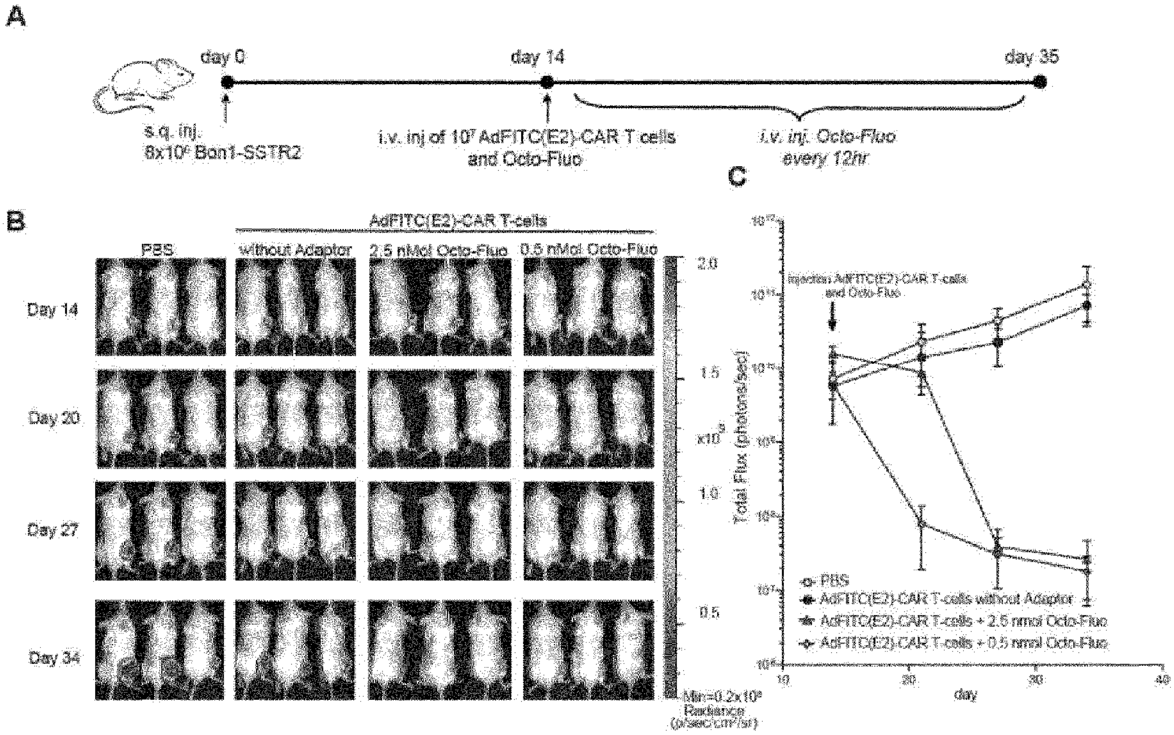
A
day 0
day 14
day 35
s.q. inj.
8x10⁶ Bon1-SSTR2
i.v. inj of 10⁷ AdFITC(E2)-CAR T cells
and Octo-Fluo
i.v. inj. Octo-Fluo
every 12hr
B
AdFITC(E2)-CAR T-cells
PBS
without Adaptor
2.5 nMol Octo-Fluo
0.5 nMol Octo-Fluo
Day 14
Day 20
Day 27
Day 34
2.0
1.5
x10⁹
1.0
0.5
Min=0.2x10⁹
Radiance
(p/sec/cm²/sr)
C
Total Flux (photons/sec)
day
PBS
AdFITC(E2)-CAR T-cells without Adaptor
AdFITC(E2)-CAR T-cells + 2.5 nmol Octo-Fluo
AdFITC(E2)-CAR T-cells + 0.5 nmol Octo-Fluo

Figure 3

D

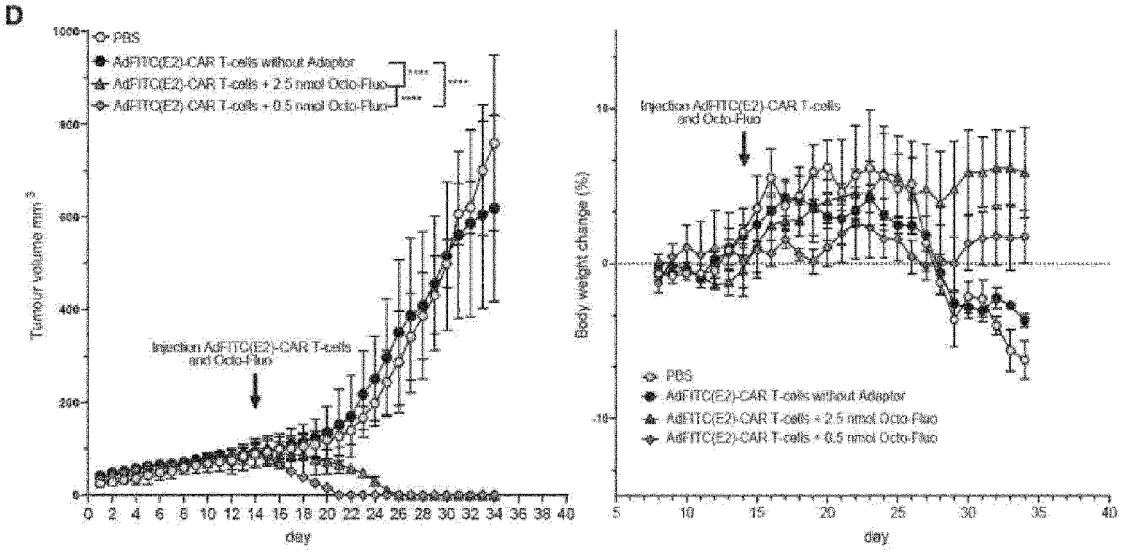
PBS
AdFITC(E2)-CAR T-cells without Adaptor
AdFITC(E2)-CAR T-cells + 2.5 nmol Octo-Fluo
AdFITC(E2)-CAR T-cells + 0.5 nmol Octo-Fluo
****
****
****
Tumour volume mm3
1000
800
600
400
200
0
Injection AdFITC(E2)-CAR T-cells and Octo-Fluo
0 2 4 6 8 10 12 14 16 18 20 22 24 26 28 30 32 34 36 38 40
day
Body weight change (%)
10
0
-10
Injection AdFITC(E2)-CAR T-cells and Octo-Fluo
PBS
AdFITC(E2)-CAR T-cells without Adaptor
AdFITC(E2)-CAR T-cells + 2.5 nmol Octo-Fluo
AdFITC(E2)-CAR T-cells + 0.5 nmol Octo-Fluo
5 10 15 20 25 30 35 40
day

Figure 4

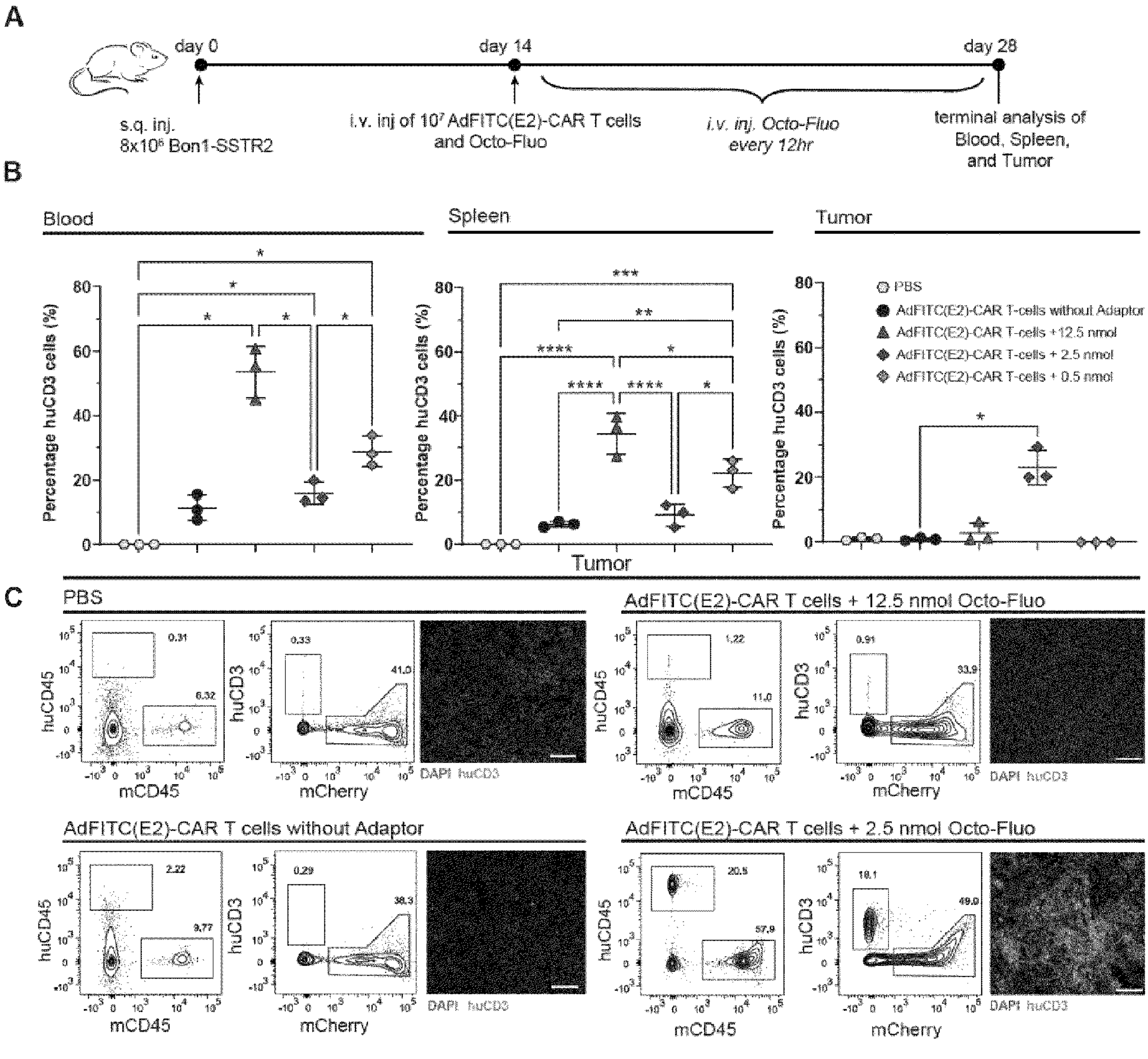
A
day 0
day 14
day 28
s.q. inj.
8x10⁶ Bon1-SSTR2
i.v. inj of 10⁷ AdFITC(E2)-CAR T cells
and Octo-Fluo
i.v. inj. Octo-Fluo
every 12hr
terminal analysis of
Blood, Spleen,
and Tumor
B
Blood
Spleen
Tumor
Percentage huCD3 cells (%)
PBS
AdFITC(E2)-CAR T-cells without Adaptor
AdFITC(E2)-CAR T-cells +12.5 nmol
AdFITC(E2)-CAR T-cells + 2.5 nmol
AdFITC(E2)-CAR T-cells + 0.5 nmol
C
PBS
AdFITC(E2)-CAR T cells + 12.5 nmol Octo-Fluo
AdFITC(E2)-CAR T cells without Adaptor
AdFITC(E2)-CAR T cells + 2.5 nmol Octo-Fluo
huCD45
mCD45
huCD3
mCherry
DAPI huCD3

Figure 5

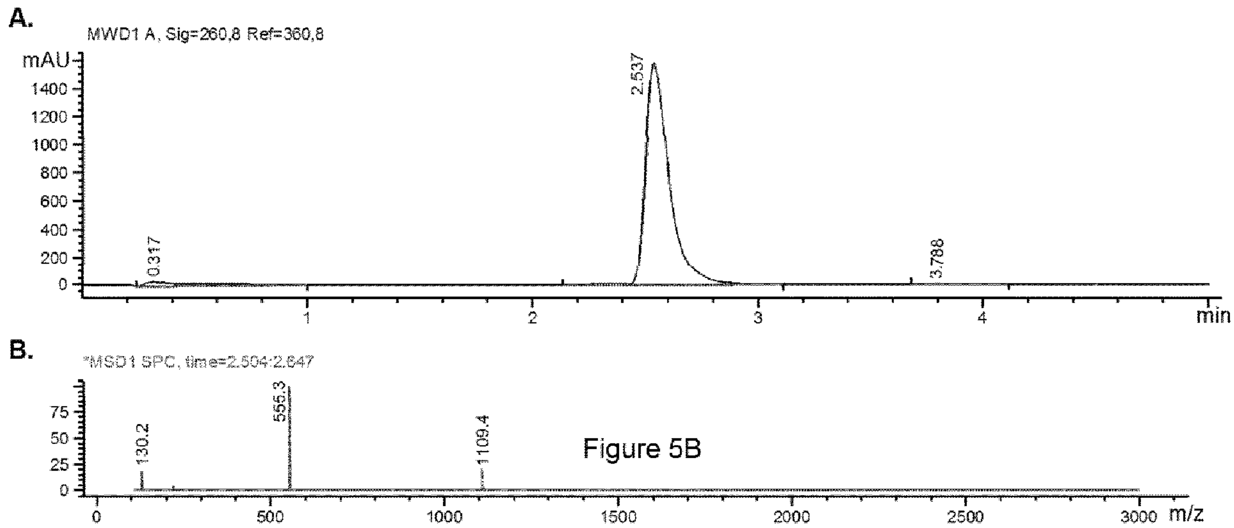
A.
MWD1 A, Sig=260,8 Ref=360,8
mAU
1400
1200
1000
800
600
400
200
0
2.537
0.317
3.788
1
2
3
4
min
B.
*MSD1 SPC, time=2.504:2.647
75
50
25
0
130.2
555.3
1109.4
Figure 5B
0
500
1000
1500
2000
2500
3000
m/z

Figure 6

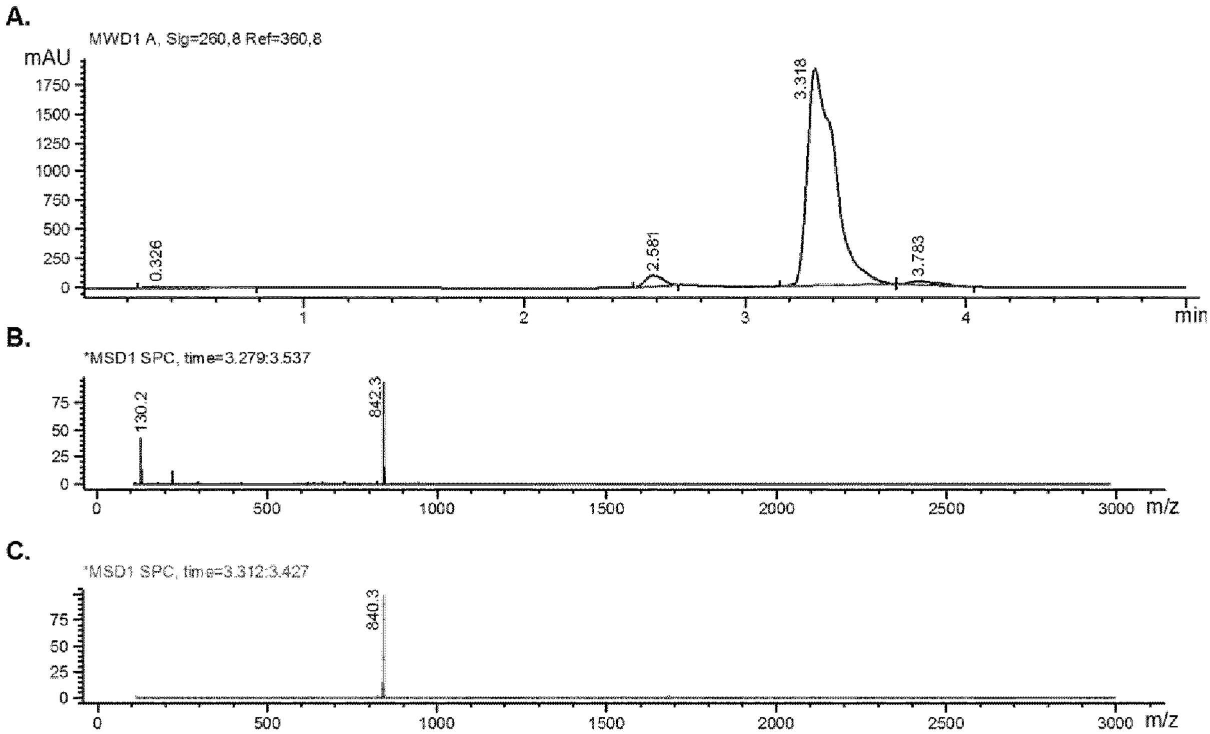
A.
MWD1 A, Sig=260,8 Ref=360,8
mAU
1750
1500
1250
1000
750
500
250
0
0.326
2.581
3.318
3.783
1
2
3
4
min
B.
*MSD1 SPC, time=3.279:3.537
75
50
25
0
130.2
842.3
0
500
1000
1500
2000
2500
3000
m/z
C.
*MSD1 SPC, time=3.312:3.427
75
50
25
0
840.3
0
500
1000
1500
2000
2500
3000
m/z

Figure 7

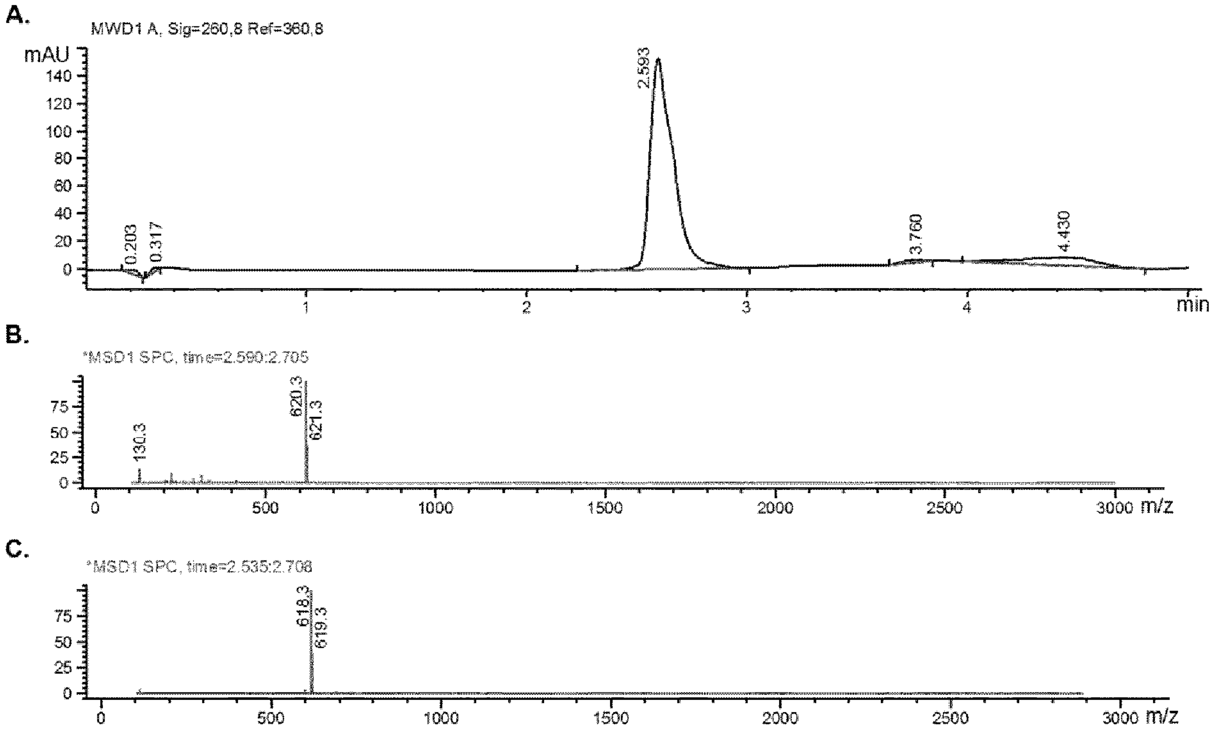
A.
MWD1 A, Sig=260,8 Ref=360,8
mAU
140
120
100
80
60
40
20
0
0.203
0.317
2.593
3.760
4.430
1
2
3
4
min
B.
*MSD1 SPC, time=2.590:2.705
75
50
25
0
130.3
620.3
621.3
0
500
1000
1500
2000
2500
3000
m/z
C.
*MSD1 SPC, time=2.535:2.708
75
50
25
0
618.3
619.3
0
500
1000
1500
2000
2500
3000
m/z

Figure 8

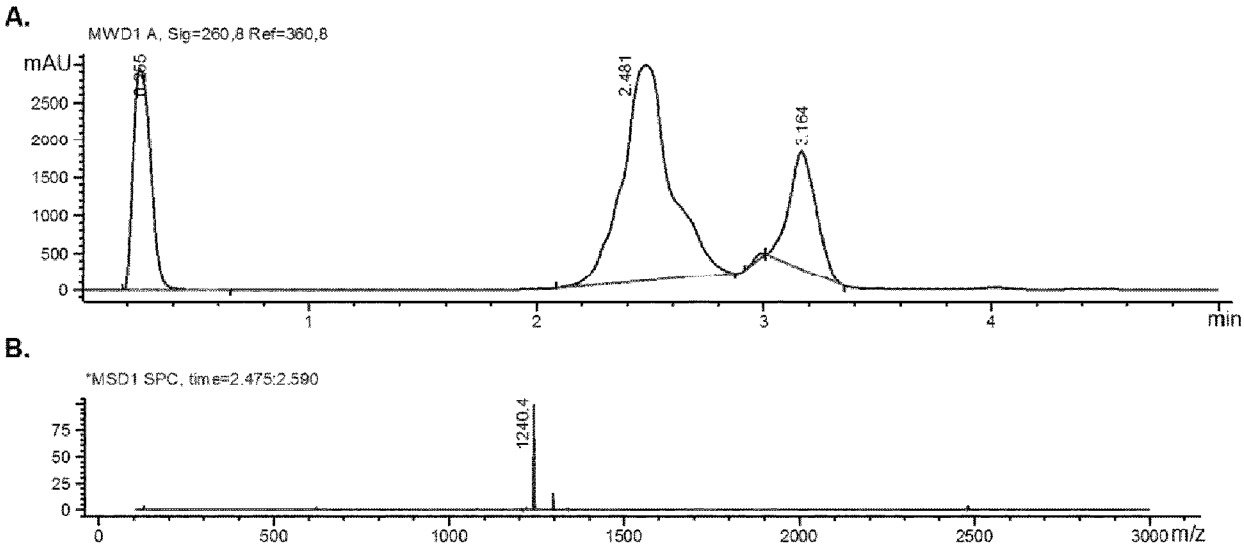
A.
MWD1 A, Sig=260,8 Ref=360,8
mAU
2500
2000
1500
1000
500
0
2.481
3.164
1
2
3
4
min
B.
*MSD1 SPC, time=2.475:2.590
75
50
25
0
1240.4
0
500
1000
1500
2000
2500
3000
m/z

Figure 9

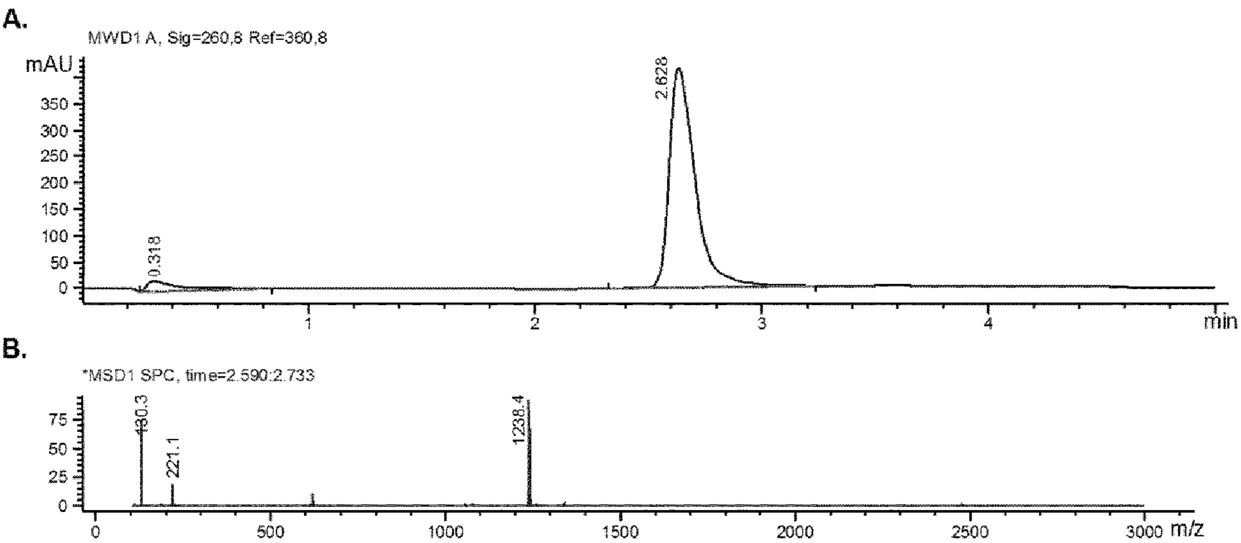
A.
MWD1 A, Sig=260,8 Ref=360,8
mAU
350
300
250
200
150
100
50
0
2.628
0.318
1
2
3
4
min
B.
*MSD1 SPC, time=2.590:2.733
75
50
25
0
130.3
221.1
1238.4
0
500
1000
1500
2000
2500
3000
m/z

Figure 10

A.

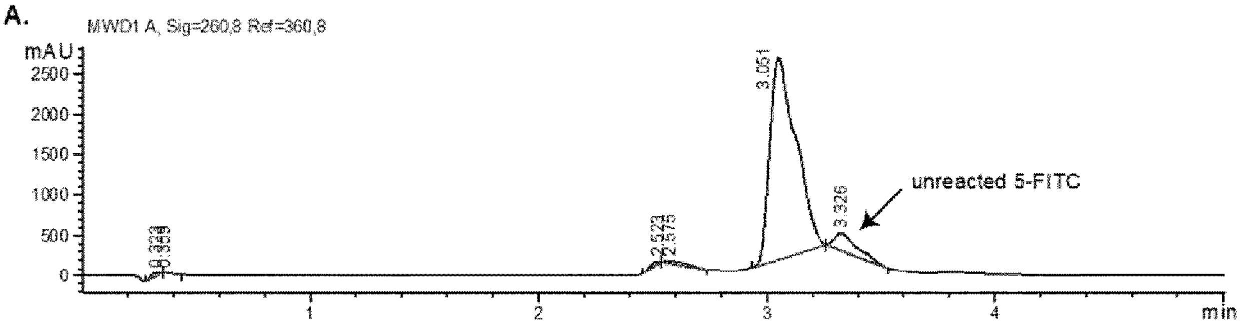
MWD1 A, Sig=260,8 Ref=360,8
mAU
2500
2000
1500
1000
500
0
3.051
3.326
2.523
2.575
unreacted 5-FITC
1
2
3
4
min

B.

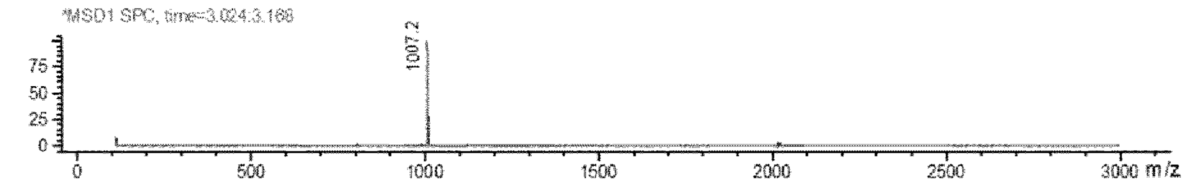
*MSD1 SPC, time=3.024:3.168
75
50
25
0
1007.2
0
500
1000
1500
2000
2500
3000
m/z

Figure 10

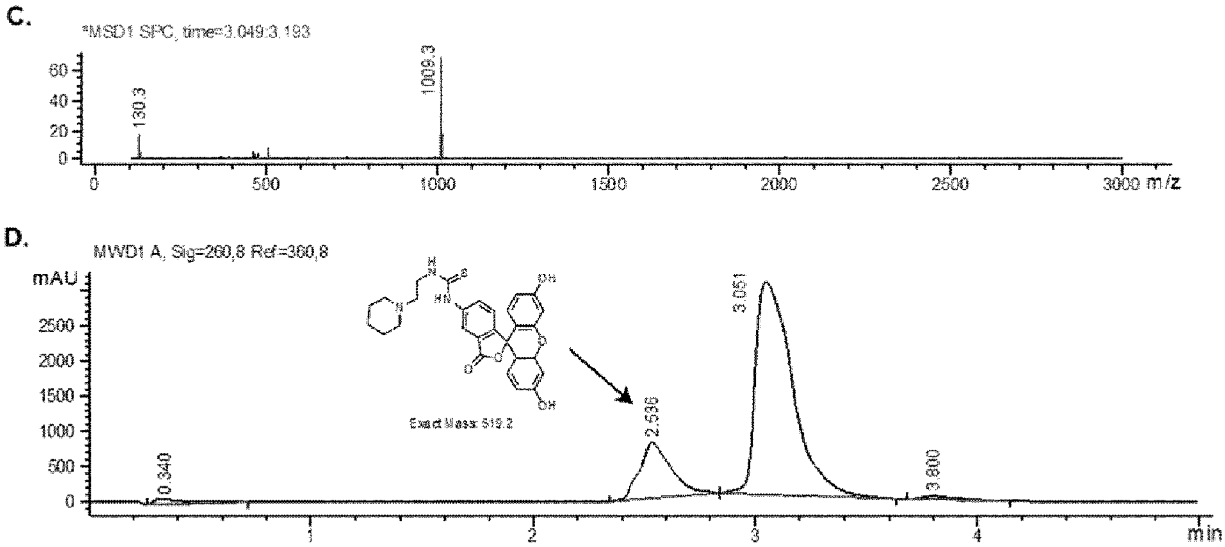
C.
*MSD1 SPC, time=3.049:3.193
1009.3
130.3
60
40
20
0
0
500
1000
1500
2000
2500
3000 m/z
D.
MWD1 A, Sig=260,8 Ref=360,8
mAU
2500
2000
1500
1000
500
0
Exact Mass: 519.2
3.051
2.536
0.340
3.800
1
2
3
4
min

Figure 11

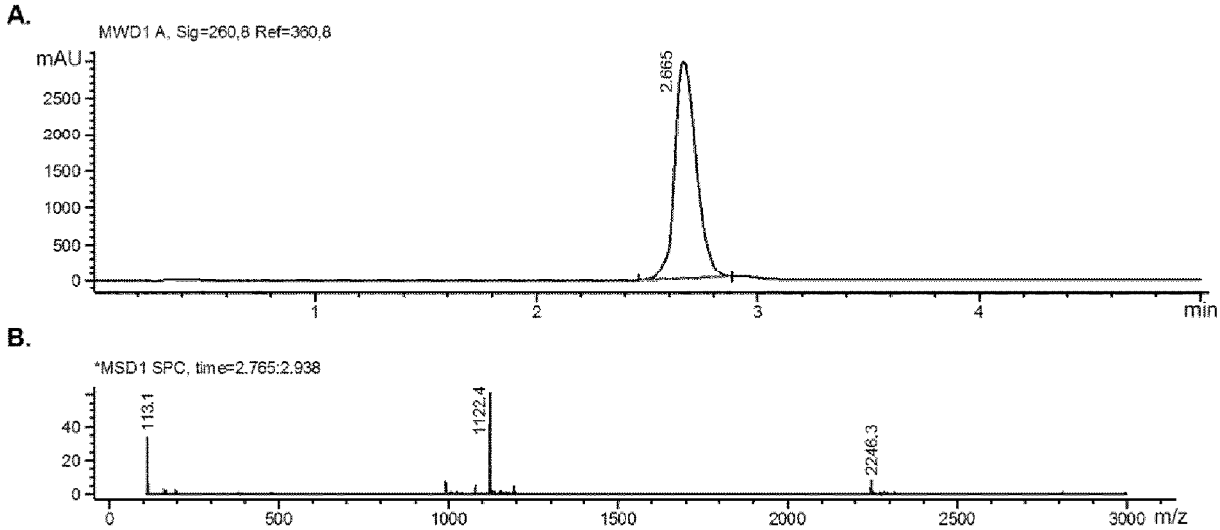
A.
MWD1 A, Sig=260,8 Ref=360,8
mAU
2500
2000
1500
1000
500
0
2.665
1
2
3
4
min
B.
*MSD1 SPC, time=2.765:2.938
40
20
0
113.1
1122.4
2246.3
0
500
1000
1500
2000
2500
3000
m/z

Figure 12

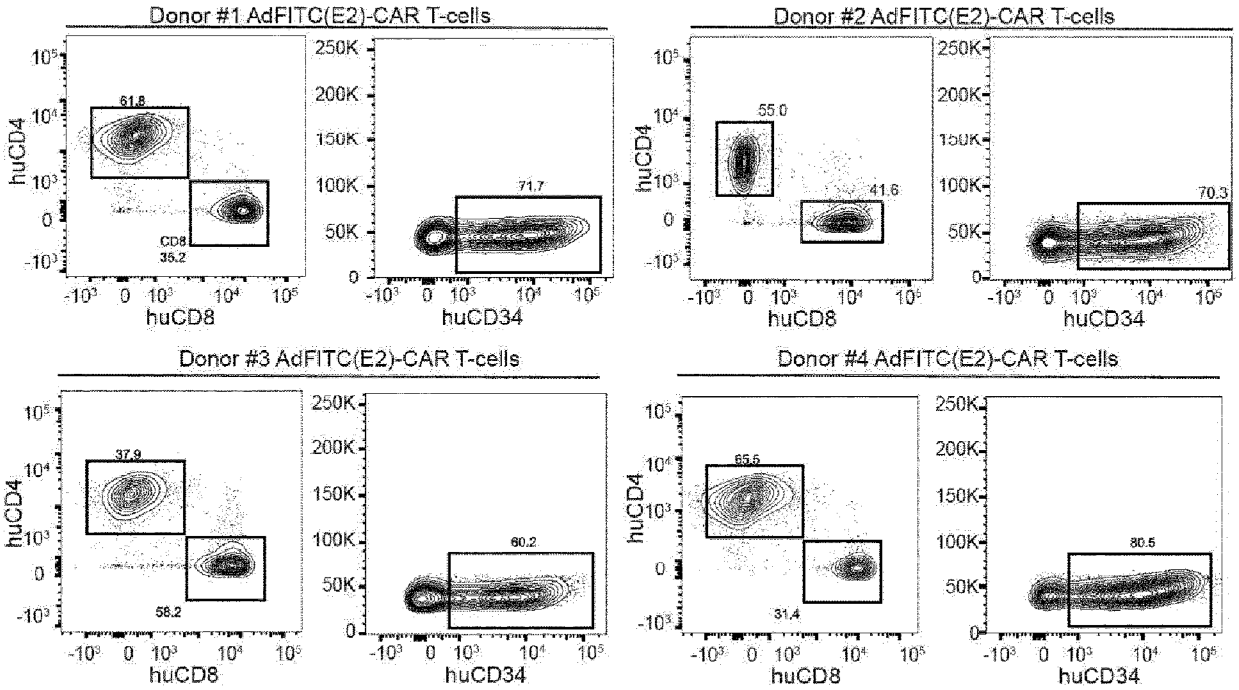
Donor #1 AdFITC(E2)-CAR T-cells
Donor #2 AdFITC(E2)-CAR T-cells
Donor #3 AdFITC(E2)-CAR T-cells
Donor #4 AdFITC(E2)-CAR T-cells
huCD4
huCD8
huCD34
61.8
CD8
35.2
71.7
55.0
41.6
70.3
37.9
58.2
60.2
65.5
31.4
80.5

Figure 13

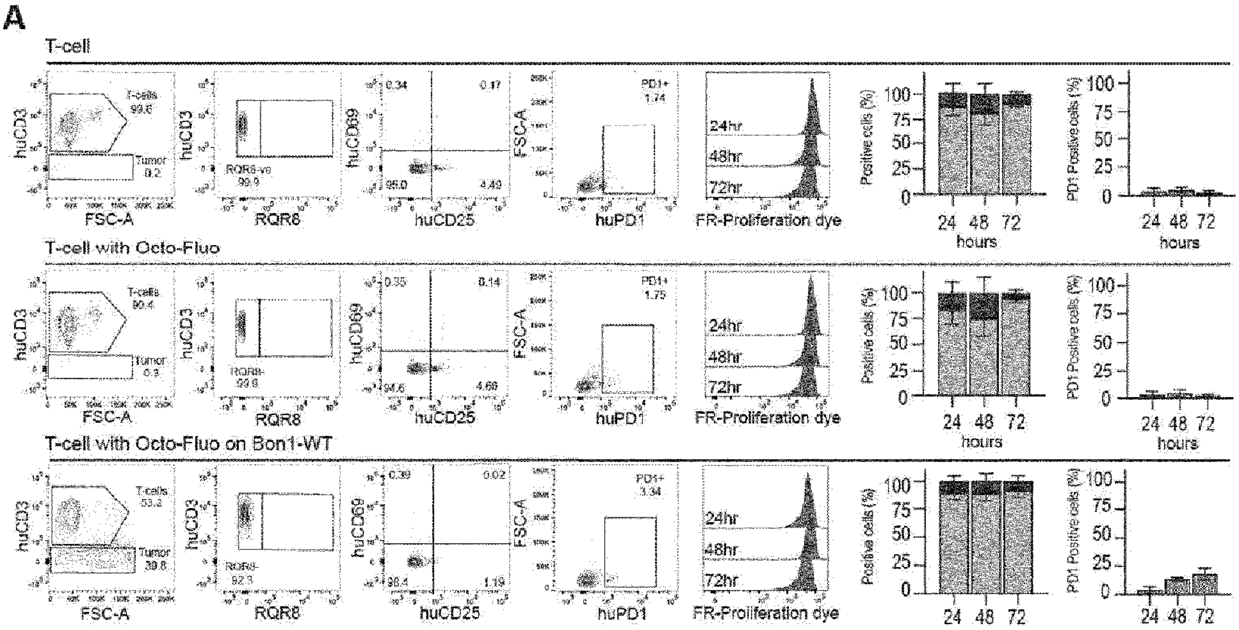
A
T-cell
T-cell with Octo-Fluo
T-cell with Octo-Fluo on Bon1-WT
huCD3
FSC-A
RQR8
huCD69
huCD25
huPD1
FR-Proliferation dye
24hr
48hr
72hr
Positive cells (%)
PD1 Positive cells (%)
hours

Figure 13

B

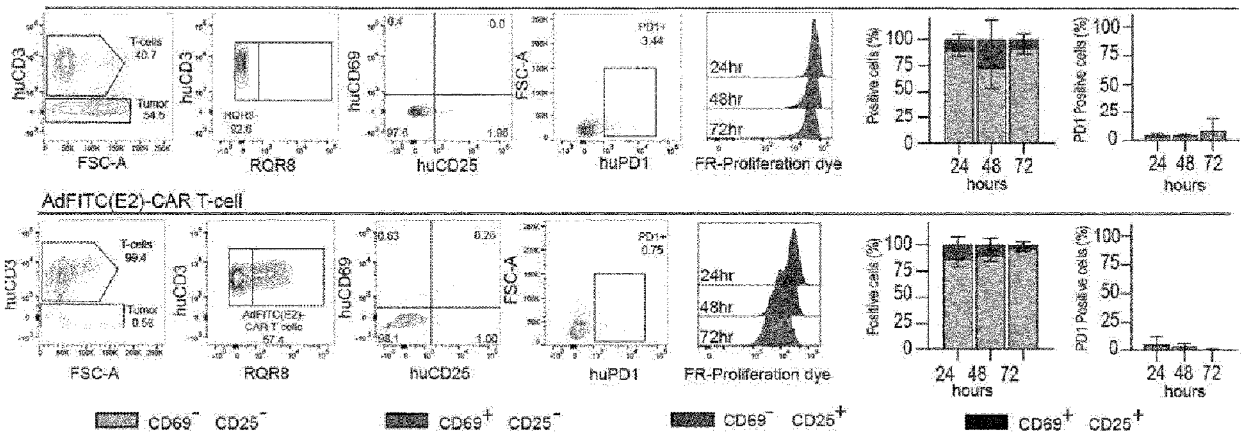
huCD3
T-cells
40.7
Tumor
54.5
FSC-A
RQR8
huCD69
huCD25
FSC-A
PD1+
3.44
huPD1
24hr
48hr
72hr
FR-Proliferation dye
Positive cells (%)
hours
PD1 Positive cells (%)
AdFITC(E2)-CAR T-cell
T-cells
99.4
Tumor
0.58
AdFITC(E2)-CAR T cells
PD1+
0.75
CD69⁻ CD25⁻
CD69⁺ CD25⁻
CD69⁻ CD25⁺
CD69⁺ CD25⁺

Figure 14

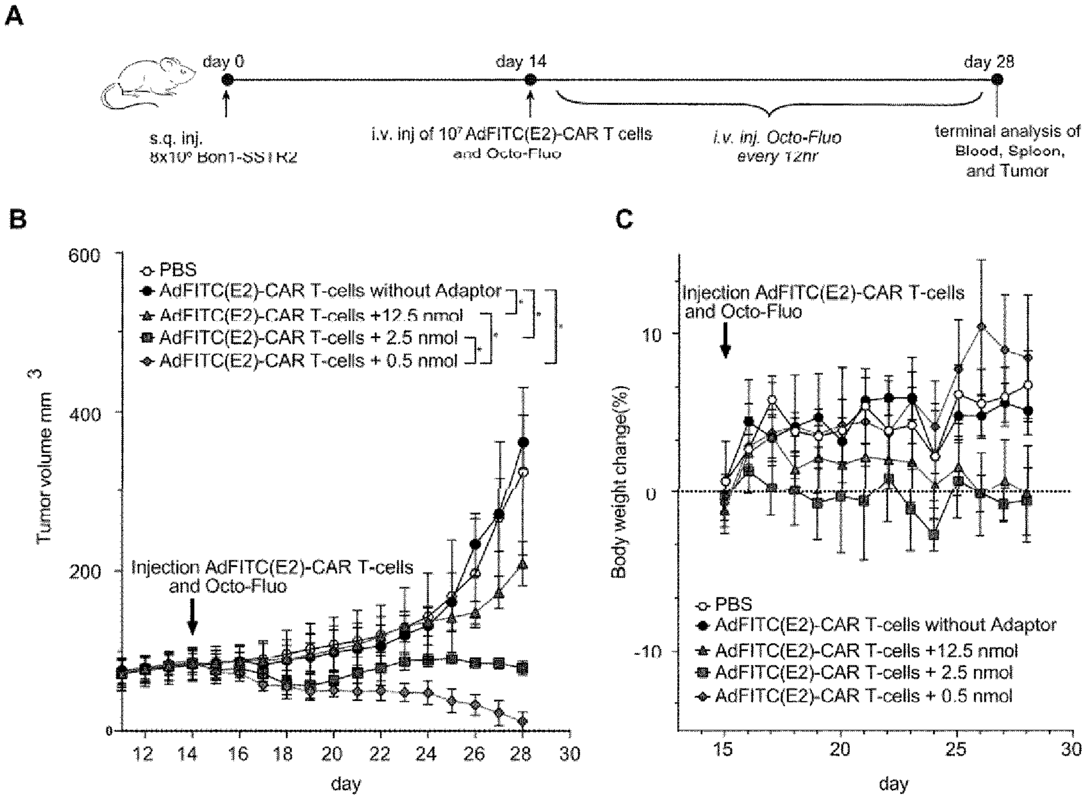
A
day 0
day 14
day 28
s.q. inj.
i.v. inj of 10$^7$ AdFITC(E2)-CAR T cells
and Octo-Fluo
i.v. inj. Octo-Fluo
every 12hr
terminal analysis of
Blood, Spleen,
and Tumor
B
Tumor volume mm 3
PBS
AdFITC(E2)-CAR T-cells without Adaptor
AdFITC(E2)-CAR T-cells +12.5 nmol
AdFITC(E2)-CAR T-cells + 2.5 nmol
AdFITC(E2)-CAR T-cells + 0.5 nmol
Injection AdFITC(E2)-CAR T-cells
and Octo-Fluo
600
400
200
0
12 14 16 18 20 22 24 26 28 30
day
C
Body weight change(%)
Injection AdFITC(E2)-CAR T-cells
and Octo-Fluo
10
0
-10
PBS
AdFITC(E2)-CAR T-cells without Adaptor
AdFITC(E2)-CAR T-cells +12.5 nmol
AdFITC(E2)-CAR T-cells + 2.5 nmol
AdFITC(E2)-CAR T-cells + 0.5 nmol
15 20 25 30
day

Figure 15

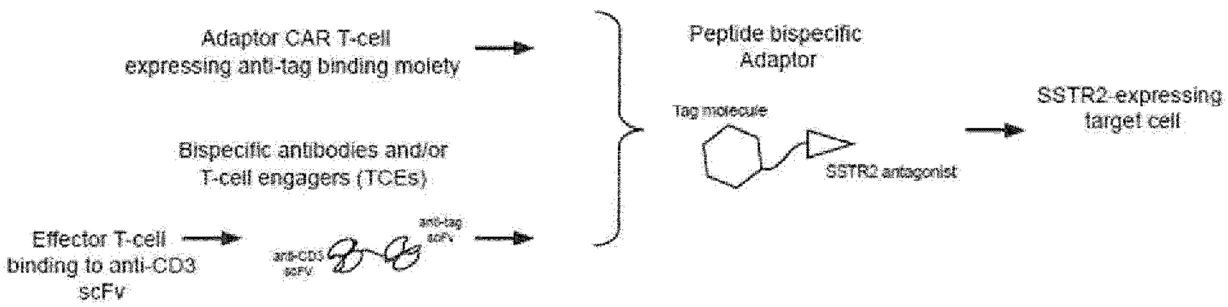
Adaptor CAR T-cell
expressing anti-tag binding moiety
Bispecific antibodies and/or
T-cell engagers (TCEs)
Effector T-cell
binding to anti-CD3
scFv
anti-CD3
scFv
anti-tag
scFv
Peptide bispecific
Adaptor
Tag molecule
SSTR2 antagonist
SSTR2-expressing
target cell

Europäisches Patentamt
European Patent Office
Office européen des brevets

# EUROPEAN SEARCH REPORT

Application Number
EP 24 20 4326

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2018/022957 A1 (TARVEDA THERAPEUTICS INC [US]) 1 February 2018 (2018-02-01) * par. 6, 24, 296 * | 1-9, 11-15 | INV. C07K7/06 A61K38/00 A61K38/12 C07K14/655 C07K14/705 |
| X<br>A | US 2019/276513 A1 (KOSTENICH GENADY [IL] ET AL) 12 September 2019 (2019-09-12) * Table 1;SEQ. 4; par. 243, 249-255, 345 * | 1-4,6,8, 9,15<br>5,7, 11-14 | |
| X<br>A | CN 102 114 247 A (UNIV CHINA PHARMA) 6 July 2011 (2011-07-06) * Example 7 * | 1-4,6,8, 9<br>5,7, 11-15 | |
| X<br>A | WO 2024/055886 A1 (JENKEM TECH CO LTD LIAONING [CN]) 21 March 2024 (2024-03-21) * par. 147-148 of the translation; p.51-55 of the Chinese language document (structures) * | 1-4,6,8, 9<br>5,7, 11-15 | |
| | | | TECHNICAL FIELDS SEARCHED (IPC) |
| | | | C07K C12N A61K |

~~The present search report has been drawn up for all claims~~

3

| Place of search | Date of completion of the search | Examiner |
|---|---|---|
| The Hague | 4 March 2025 | Hohwy, Morten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

Application Number
EP 24 20 4326

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-9, 11-15(all partially)

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 24 20 4326

The Search Division considers that the present European patent application does not comply with the requirements of unity of invention and relates to several inventions or groups of inventions, namely:

1. claims: 1-9, 11-15(all partially)

An adaptor molecule according to claim 1 and uses hereof, wherein the SSTR2 targeting moiety is Lanreotide.

---

2. claims: 1-9, 11-15(all partially)

An adaptor molecule according to claim 1 and uses hereof, wherein the SSTR2 targeting moiety is Octeotide.

---

3. claims: 1-9, 11-15(all partially)

An adaptor molecule according to claim 1 and uses hereof, wherein the SSTR2 targeting moiety is Pasireotide.

---

4. claims: 10(completely); 1-9, 11-15(partially)

An adaptor molecule according to claim 1 and uses hereof, wherein the SSTR2 targeting moiety is is structure (II) of claim 4 (Bass).

---

## ANNEX TO THE EUROPEAN SEARCH REPORT ON EUROPEAN PATENT APPLICATION NO.

EP 24 20 4326

This annex lists the patent family members relating to the patent documents cited in the above-mentioned European search report. The members are as contained in the European Patent Office EDP file on
The European Patent Office is in no way liable for these particulars which are merely given for the purpose of information.

04-03-2025

| Patent document cited in search report | Publication date | Patent family member(s) | Publication date |
|---|---|---|---|
| WO 2018022957 A1 | 01-02-2018 | NONE | |
| US 2019276513 A1 | 12-09-2019 | EP 3166967 A1 | 17-05-2017 |
| | | ES 2942260 T3 | 31-05-2023 |
| | | PL 3166967 T3 | 24-07-2023 |
| | | US 2017298113 A1 | 19-10-2017 |
| | | US 2019276513 A1 | 12-09-2019 |
| | | US 2020325203 A1 | 15-10-2020 |
| | | US 2022162284 A1 | 26-05-2022 |
| | | WO 2016038565 A1 | 17-03-2016 |
| CN 102114247 A | 06-07-2011 | NONE | |
| WO 2024055886 A1 | 21-03-2024 | CN 115505028 A | 23-12-2022 |
| | | WO 2024055886 A1 | 21-03-2024 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *CHEMICAL ABSTRACTS*, 118488-18-9
- *CHEMICAL ABSTRACTS*, 103213-32-7
- *CHEMICAL ABSTRACTS*, 71989-35-0
- *CHEMICAL ABSTRACTS*, 71989-26-9
- *CHEMICAL ABSTRACTS*, 163619-04-3
- *CHEMICAL ABSTRACTS*, 71989-38-3
- *CHEMICAL ABSTRACTS*, 167015-11-4
- *CHEMICAL ABSTRACTS*, 95753-55-2
- *CHEMICAL ABSTRACTS*, 129460-09-9
- *CHEMICAL ABSTRACTS*, 18523-48-3
- *CHEMICAL ABSTRACTS*, 3326-32-7
- *CHEMICAL ABSTRACTS*, 27578-60-5
- *CHEMICAL ABSTRACTS*, 121-44-8
- *CHEMICAL ABSTRACTS*, 7087-68-5
- *CHEMICAL ABSTRACTS*, 148893-10-1